**Europäisches Patentamt**

**European Patent Office**

**Office européen des brevets**

(19)

(11) Veröffentlichungsnummer: **0 145 005 B1**

(12) # EUROPÄISCHE PATENTSCHRIFT

(45) Veröffentlichungstag der Patentschrift:
**12.04.89**

(51) Int. Cl.⁴: **A 61 K 35/42, A 61 K 37/22**

(21) Anmeldenummer: **84115103.8**

(22) Anmeldetag: **10.12.84**

(54) **Lungen-Surfactant, Verfahren zu seiner Herstellung und seine pharmazeutische Verwendung.**

(30) Priorität: **08.12.83 DD 257641**
**08.12.83 DD 257642**
**01.11.84 DD 268981**

(43) Veröffentlichungstag der Anmeldung:
**19.06.85 Patentblatt 85/25**

(45) Bekanntmachung des Hinweises auf die Patenterteilung:
**12.04.89 Patentblatt 89/15**

(84) Benannte Vertragsstaaten:
**AT BE CH DE FR GB IT LI NL SE**

(56) Entgegenhaltungen:
**EP-A- 0 055 041**
**DE-A- 2 335 334**
**GB-A- 2 050 832**
**US-A- 4 397 839**

(73) Patentinhaber: **VEB Arzneimittelwerk Dresden,**
**Wilhelm-Pieck-Strasse 35, DDR-8122 Radebeul 1 (DD)**

(72) Erfinder: **Lachmann, Burkhardt Dr.sc.med.Dr.Med.Sci.,**
**Spinolastrasse 32, D-1116 Berlin-Karow (DD)**
Erfinder: **Rattke, Wilfried, Dr.rer.nat.,**
**Karl-Marx-Strasse 7, D-8122 Radebeul (DD)**
Erfinder: **Boyde, Peter, Dr.rer.nat., Wieckestrasse 4,**
**D-8036 Dresden (DD)**
Erfinder: **Pötter, Heinrich, Dr.rer.nat., Finstere Gasse 14,**
**D-8122 Radebeul (DD)**
Erfinder: **Dauth, Christoph, Dr.rer.nat.,**
**Scheringerstrasse 37, D-8122 Radebeul (DD)**
Erfinder: **Draheim, Regina, Dipl.-Biochem.,**
**Wasastrasse 19/7, D-8122 Radebeul (DD)**
Erfinder: **Illhardt, Rolf, Dr.rer.nat.,**
**Bernhard-Voss-Strasse 24, D-8122 Radebeul (DD)**

(74) Vertreter: **Patentanwälte Beetz sen. - Beetz jun. Timpe -**
**Siegfried - Schmitt-Fumian, Steinsdorfstrasse 10,**
**D-8000 München 22 (DE)**

## Beschreibung

Die Erfindung bezieht sich auf eine Lungen-Surfactantpräparation, Verfahren zu seiner Herstellung sowie pharmazeutische Mittel, die sich insbesondere zur Behandlung des durch Surfactantmangel in der Lunge bedingten Atemnotsyndroms bei Patienten mit entweder noch nicht ausgebildeter oder pathologisch veränderter Surfactantschicht. Als Ausgangsmaterial werden Lungen von Säugetieren herangezogen. Der von den Alveolarzellen des Typs II gebildete Wirkstoff kann zur kausalen Therapie des durch Surfactantmangel bedingten Atemnotsyndroms eingesetzt werden.

Die Hyalinmembran-Erkrankung (HMD) beruht auf einem Mangel an Alveolar-Surfactant in der Lunge und bedingt dadurch die Ausbildung von Atelektasen. Eine medikamentöse und kausale Behandlungsmöglichkeit wird in der Verabreichung von Lungen-Surfactant in die Trachea gesehen. Tierexperimentelle und erste klinische Versuche an Neugeborenen wurden sowohl mit natürlichem Surfactant als auch mit dessen Hauptbestandteil Dipalmityllecithin, vorwiegend in Kombination mit Phosphatidylglycerin, durchgeführt. Das natürliche Surfactant wird von einigen Autoren (Morley, C.N., et al., The Lancet 1 (1981) 64–68) wegen ungenügender und stark variierender Aktivität als für einen therapeutischen Einsatz ungeeignet angesehen. Es wurde bereits ein halbsynthetisches Surfactant entwickelt, das aus einem Gemisch von natürlich vorkommendem Surfactant aus Säugerlungen und synthetischen Phospholipiden besteht. An 10 unreifen Frühgeborenen mit HMD wurden gute Behandlungserfolge erzielt (Fujiwara, T., et al., The Lancet 1 (1980) 55–59).

Die Gewinnung von Lungen-Surfactant kann aus Lungenhomogenisat (V. Neergard, K., Verh. Dtsch. Ges. Inn. Med. 41 (1929) 249; Clements, J.A., Proc. Sec. exp. Biol. (N.Y.) 95 (1957) 170), durch pervasale Ausspülung (Bondurant, S., Miller, D.A., J. Appl. Physiol. 17 (1962) 167) oder broncheale Spülung (Clements, J.A., Proc. Soc. exp. Biol. (N.Y.) 95 (1957) 170) erfolgen. Für eine therapeutische Nutzung des Wirkstoffs kommt nur die Isolierung aus stark zerkleinertem Lungengewebe in Betracht. Der dabei gewonnene Extrakt enthält allerdings nicht nur die Komponenten des grenzflächenaktiven Systems, sondern auch, in Abhängigkeit von den angewandten Extraktionsbedingungen, lösliche und emulgierbare Bestandteile der Lunge und des in der Lunge enthaltenen Bluts.

Eine Möglichkeit zur Gewinnung dieses grenzflächenaktiven Materials zur Behandlung der HMD ist in der DE-A1-3 021 006 beschrieben. Durch Elektrolytlösung wird aus Säugerlunge ein rohes Material gewonnen, dem zur Erreichung eines Phospholipidgehalts von 75 bis 95,5% die erforderliche Menge synthetisches Phospholipid zugesetzt wird. Der Proteingehalt beträgt 0,5 bis 5,0%. Nach dem Abzentrifugieren von Zellfragmenten bei einer Drehzahl von 500 bis 1500 $min^{-1}$ wird das rohe Wirkstoffgemisch durch Zentrifugieren bei einer Drehzahl von 12 000 bis 16 000 $min^{-1}$ und 0 bis 10 °C als Sediment erhalten. Die weitere Aufarbeitung erfolgt durch mehrfache Wiederaufnahme der Lipide in Natriumchloridlösung und Abtrennung durch hochtouriges Zentrifugieren. Nach Dialyse, Extraktion mit Aceton und Aufnahme der Phospholipide in einem Chloroform-Methanol-Gemisch sowie Abdampfen des Lösungsmittels wird ein Wirkstoffgemisch erhalten, dem dann in Bezug auf den Phosphatgehalt im molaren Verhältnis 1:0,65:0,12 Dipalmityllecithin und Phosphatidylglycerol zugesetzt werden.

Auch andere in der Literatur beschriebene Verfahren (Brown, E.S., et al., J. Appl. Physiol. 14 (1959) 717; Jobe, A., Biochem. Biophys. Acta 489 (1977) 440–453; King, R.J., Clements, J.A., Am. J. Physiol, 223 (1972) 707–714; Bergren, P., et al., Ircs Med. Sci. 9 (1981) 283–284) zur Gewinnung von natürlichem Surfactant basieren auf der Extraktion oder dem Spülen von Säugerlunge mit Elektrolytlösungen und der anschließenden Trennung durch mehrfaches Zentrifugieren bei unterschiedlichen relativen Zentrifugalbeschleunigungen und Temperaturen, wobei die Abtrennung der Phospholipide die Anwendung hoher relativer Zentrifugalbeschleunigungen und Dichtegradienten erfordert, was bei der Maßstabsvergrößerung einen erheblichen Aufwand mit sich bringt.

Die Gewinnung eines Roh-Surfactans durch Extraktion von Hunde- und Kaninchenlunge mit isotonischer Natriumchloridlösung und anschließendes mehrstufiges Zentrifugieren ist ebenfalls versucht worden. (Scarpelli, E.M., et al., J. Appl. Physiol. 23 (1967) 880–886). Für analytische Zwecke wurden die Bestandteile des Surfactants durch Gelfiltration an Sephadex[®] G 200 getrennt. Aufgrund der unterschiedlichen Partikelgrößen und der grenzflächenaktiven Eigenschaften führt die Gelfiltration zu hohen Materialverlusten und erscheint schon deshalb für präparative Zwecke nicht geeignet.

Ein synthetisches Lungen-Surfactant aus einem Gemisch von Dipalmityl-Lecithin und Phosphatidylglycerol im Verhältnis 7:3 erwies sich bei Neugeborenen mit HMD als nicht ausreichend wirksam (Morley, C.J., et al., The Lancet 1 (1981) 64–68). Die Herstellung eines synthetischen, proteinfreien Surfactants aus Dipalmityllecithin und Hexadecanol ist ferner in der US-A-4 312 600 beschrieben.

Der Erfindung liegt die Aufgabe zugrunde, eine Lungen-Surfactantpräparation und ihre Herstellung sowie entsprechende pharmazeutische Mittel mit diesem Lungen-Surfactant als Wirkstoff anzugeben, mit dem eine Substitutionstherapie des bei Surfactantmangel in der Lunge auftretenden Atemnotsyndroms möglich ist. Das Herstellungsverfahren soll dabei einfach im großtechnischen Maßstab mit leicht zugänglichen Hilfsstoffen auf der Basis natürlicher Rohstoffe durchführbar sein.

Die Aufgabe wird anspruchsgemäß gelöst. Vorteilhafte Weiterbildungen sind Gegenstand der abhängigen Ansprüche.

Das erfindungsgemäße Verfahren zur Herstellung des Lungen-Surfactants ist gekennzeichnet durch

(A)
(A1) Extraktion von zerkleinertem Lungengewebe oder eines wäßrigen, mit einer isotonischen Lösung eines Elektrolyts oder eines Mono- oder Disaccharids hergestellten Lungenextrakts mit einem nicht mit Wasser mischbaren Lipidlösungsmittel,
(A2) Abtrennung ungelöster Bestandteile aus dem Extrakt,
(A3) Abtrennung des Lipidlösungsmittels,
(A4) Extraktion mit einem wasserlöslichen Alkohol,
(A5) Abtrennung alkoholunlöslicher Bestandteile aus dem Extrakt
und
(A6) Abtrennung des Alkohols und Gewinnung des Wirkstoffs
oder

(B)
(B1) Abzentrifugieren der Zellfragmente aus einem wäßrigen, mit einer isotonischen Lösung eines Elektrolyts oder eines Mono- oder Disaccharids hergestellten Lungenextrakts bei niederen relativen Zentrifugalbeschleunigungen,
(B2) Inkontaktbringen des Extrakts mit einem unlöslichen Adsorbens,
(B3) Waschen des beladenen Adsorbens mit Wasser oder Aceton unter Entfernung von nicht adsorbiertem Material,
(B4) Desorption des Wirkstoffs mit einem organischen Lösungsmittel oder einem Lösungsmittelgemisch
und
(B5) Abtrennung des bzw. der Lösungsmittel unter Gewinnung des Wirkstoffs.

Die erfindungsgemäße Lungen-Surfactantpräparation ist entsprechend nach diesem Verfahren erhältlich.

Die pharmazeutischen Mittel enthalten dieses Lungen-Surfactant neben üblichen Hilfs- und/oder Trägerstoffen. Der Träger ist dabei vorzugsweise flüssig, wobei bevorzugt physiologische Flüssigkeiten bzw. Lösungen verwendet werden, und galenisch so formuliert, daß das Mittel über die Atemwege verabreicht werden kann.

Mit dem erfindungsgemäß zugänglichen, dem natürlichen entsprechenden Lungen-Surfactant spezieller Zusammensetzung ist eine kausale Therapie aller Erkrankungen möglich, die mit einer Veränderung des pulmonalen Surfactants einhergehen, insbesondere des Atemnotsyndroms bei Neugeborenen und Erwachsenen. Solche Therapeutica besitzen außerordentliche medizinische und humanitäre Bedeutung, vor allem im Hinblick auf die Verringerung der Säuglingssterblichkeit bei Neugeborenen, deren Surfactantschicht noch nicht ausgebildet ist, sowie die Notfalltherapie bei Patienten, deren Lungen-Surfactant durch pathologische Vorgänge geschädigt worden ist.

Die Erfindung beruht auf der überraschenden Feststellung, daß der phospholipidreiche und proteinhaltige Surfactant-Komplex aus dem wäßrigen Extrakt von zerkleinerter Säugetierlunge oder der wäßrigen Phase der Lunge selbst in ein einheitliches, nicht mit Wasser mischbares Lipidlösungsmittel, wie z.B. Chloroform, Dichlormethan, Tetrachlormethan oder Petrolether, übergeführt werden kann, ohne dabei seine biologische Wirksamkeit zu verlieren. Ein großer Teil von störenden Zellbestandteilen, insbesondere Proteine, fällt dabei aus und kann leicht abgetrennt werden. Das bisher zum Lösen des Surfactants hauptsächlich für analytische Zwecke verwendete Extraktionsmittel nach Folch (Chloroform/Methanol im Volumenverhältnis 2:1) wird somit durch ein einheitliches Lipidlösungsmittel ersetzt, wobei ein Alkoholanteil vermieden ist. Bevorzugt werden Chloroform sowie andere halogenierte Kohlenwasserstoffe. Hierdurch kann wiederum die bei den bisher üblichen Laborverfahren angewandte Vielzahl von Zentrifugierschritten, zum Teil bei sehr relativer Zentrifugalbeschleunigung und unter Anwendung von Dichtegradienten, vermieden werden. Der Wegfall eines Alkoholzusatzes zum Lösungsmittel hat den Vorteil einer erheblichen Reduzierung des Proteinanteils, der infolge seiner Artfremdheit bei der Verabreichung solcher Präparate an Menschen zu allergischen Reaktionen führen kann (Bergmann, K.-Ch., Lachmann, B., Z. Erkr. Atm. 137 (1972) 55–60; Morley, C.N., et al., The Lancet 1 (1981) 64–68). Andererseits geht der zur Entfaltung der Wirksamkeit notwendige, jedoch geringe Lipoproteinanteil bei der erfindungsgemäßen Extraktion in die Lösungsmittelphase über. Darüber hinaus erleichtert die Anwendung eines einheitlichen Lipidlösungsmittels die Rückgewinnung des Lösungsmittels durch Redestillation gegenüber der Verwendung eines Gemisches verschiedener Lösungsmittel und trägt somit erheblich zur Verbesserung der Wirtschaftlichkeit des Verfahrens bei.

Bei der Durchführung des erfindungsgemäßen Verfahrens wird die Säugetierlunge, vorwiegend Rinder- oder Schweinelunge, von Fettgewebe und Trachearesten befreit und z.B. mit einem Fleischwolf zerkleinert. Der so erhaltene Brei aus Lungengewebe wird entweder sofort mit einem Lipidlösungsmittel, wie Chloroform oder einem anderen halogenierten Kohlenwasserstoff, ausgerührt. Alternativ kann von dem Brei aus Lungengewebe ein wäßriger Extrakt, vorteilhafterweise mit isotonischen Elektrolyt- und Zuckerlösungen, hergestellt werden, der mit einem Lipidlösungsmittel, wie Chloroform oder einem anderen halogenierten Kohlenwasserstoff, ausgerührt wird. Die direkte Extraktion der Lunge mit einem Lipidlösungsmittel, wie halogenierten Kohlenwasserstoffen, führt man mit 0,5 bis 10 l Lösungsmittel und vorteilhaft 1,5 l Lösungsmittel pro Kilogramm Lungengewebe durch.

Die Herstellung eines wäßrigen Extrakts erfolgt mit 0,1 bis 0,5 kg Lungengewebe pro Liter Lösung;

die Extraktion des Surfactants aus 1 l dieser Lösung ist mit 0,1 bis 2,0 l und vorteilhafterweise 0,25 l Lipidlösungsmittel, vorzugsweise halogenierten Kohlenwasserstoffen, möglich. Die Lungenrückstände werden jeweils durch Filtration über Gaze von den Extrakten getrennt; die nach der Chloroformbehandlung des wäßrigen Extrakts ausgefallenen Proteine werden vorteilhaft durch Zentrifugieren abgetrennt. Die erhaltene Lipidlösungsmittelphase wird nach Klärung durch Filtration bei 40°C im Vakuum aufkonzentriert; anschließend kann das Lösungsmittel durch Stickstoffeinleitung bei 40°C völlig ausgetrieben werden, so daß eine pastose, gelblichbräunliche Substanz zurückbleibt. Ihr Phospholipidgehalt beträgt bei direkter Chloroformextraktion 20 bis 50%, überwiegend 30 bis 40%, und bei Zwischenschaltung der wäßrigen Extraktion 30 bis 75%, überwiegend 50 bis 60%. Der Proteingehalt des so erhaltenen, biologisch bereits gut wirksamen Rohsurfactants liegt bei direkter Chloroformextraktion zwischen 0,8 und 1,5% und bei Zwischenschaltung der wäßrigen Extraktion unter 1%, überwiegend bei 0,5%.

Das gewonnene Surfactant wird dann einer anschließenden Alkoholextraktion unterworfen. Dazu wird das rohe Surfactant im Temperaturbereich zwischen −10 und +60°C mit einem hochtourigen Rührwerk ausgerührt, wobei auf 1 g Surfactant 1 bis 20 ml und vorzugsweise 3 ml Alkohol eingesetzt werden. Als Lösungsmittel sind Methanol oder Ethanol verwendbar. Die alkoholische Phase wird durch Filtration oder Zentrifugieren abgetrennt, worauf das Lösungsmittel abgedampft wird.

Die so erhaltene trockene Substanz weist folgende Zusammensetzung auf

|  | (%) |
|---|---|
| Phospholipide | 45–65; |
| Neutrallipide | 10–25; |
| Cholesterin | 10–25; |
| Proteine | 0,5–1,5; |
| freie Fettsäuren | 2–5; |

sie ist bei intratrachealer Applikation ausnahmslos gut verträglich.

Die Phospholipide liegen dabei in folgenden Mengenverhältnissen vor:

|  | (%) |
|---|---|
| Phosphatidylcholin | 50–60 |
| Phosphatidylglycerin | 2–3 |
| Phosphatidylethanolamin | 10–20 |
| Phosphatidylserin | 1–2 |
| Sphingomyelin | 10–25 |
| Phosphatidylinosit | 0,5–1. |

Es wurde weiterhin gefunden, daß die alkoholische Extraktion der Surfactant-Substanz günstig mehrfach vorgenommen werden kann. Hierzu wird der in Alkohol unlösliche Rückstand erneut unter den für die Extraktion angeführten Bedingungen mit Ethanol oder Methanol ausgerührt. Mit dem nun zurückbleibenden unlöslichen Material kann eine dritte Ausrührung analog zur ersten und zweiten durchgeführt werden. Das erhaltene Material hat im wesentlichen die Zusammensetzung des ersten Extrakts, jedoch nimmt der Phospholipidgehalt nach der 3. Extraktion drastisch ab, während der Cholesterolgehalt in der Folge der Extraktionsschritte ständig ansteigt. Mit der 2. und 3. Extraktion läßt sich jedoch noch gut wirksames und bei intratrachealer Instillation ausnahmslos verträgliches Material herstellen.

Es wurde ferner gefunden, daß durch Bindung des aus Lunge extrahierten Surfactans an ein unlösliches Adsorbens, insbesondere Kieselgel oder aktivierte Silicate, und anschließende Elution mit für Phospholipide geeigneten Lösungsmitteln auf einfache Weise ein Surfactantpräparant gewonnen werden kann, das eine hohe pharmakologische Wirksamkeit bei der Behandlung des Atemnotsyndroms aufweist.

Bei der Durchführung dieses erfindungsgemäßen Verfahrens erfolgt die Extraktion des Surfactants aus zerkleinerter Säugerlunge, vorzugsweise Rinder- oder Schweinelunge, durch Lösungen von Mono- oder Disacchariden, vorzugsweise durch isotonische Glucose- oder Saccharoselösung, oder, wie literaturbekannt, mit Elektrolytlösungen, z.B. isotonischer Natriumchloridlösung. Nach Abtrennung von Zellfragmenten durch Zentrifugieren bei niedrigen relativen Zentrifugalbeschleunigungen in bekannter Weise wird das Wirkstoffgemisch an Kieselgel oder aktiviertem Magnesiumsilicat adsorbiert. Die reversible Bindung von wirksamem Surfactant an Kieselgel war nicht zu erwarten, da in der Literatur ausdrücklich darauf hingewiesen wird, daß die physikalischen und chemischen Eigenschaften dieses Vielkomponentengemisches eine irreversible Bindung an feste Chromatographiematerialien bewirken und somit eine Isolierung von biologisch aktivem Surfactant auf diese Weise nicht durchführbar ist (King, R.J., Federation Proceedings 33 (1974) 2238–2247). Nach Auswaschen von nicht adsorbiertem Material mit destilliertem Wasser oder Aceton wird der aktive Wirkstoff mit einem Lösungsmittel bzw. Lösungsmittelgemisch, vorzugsweise nach Folch mit Chloroform/Methanol, vom Adsorbens desorbiert und durch Eindampfen im Vakuum oder Trocknung durch Einleiten von Stickstoff gewonnen.

Hierbei wird ein dem natürlichen entsprechendes Surfactant mit einem Phospholipidgehalt zwischen 30 und 70% und vorzugsweise 40 bis 50% erhalten. Das Präparat zeichnet sich durch einen niedrigen Proteingehalt (≤ 1,5%) aus. Der niedrige Proteingehalt ist im Hinblick auf mögliche Nebenwirkungen von Surfactant-Präparaten bedeutsam, da durch den Extraktionsvorgang sowohl Serumproteine als auch organspezifische Proteine in den Extrakt gelangen, die bei Verabreichung an Menschen antigen wirken können (Bergmann, K.-Ch., Lachmann, B., Z. Erkr. Atm. 137 (1972) 55–60; Morley, C.N., et al., The Lancet 1 (1981) 64–68).

Zur Analyse wurden folgende analytischen Methoden angewandt:

Der Phospholipidgehalt wurde nach einem von Bartlett (N. Zöllner und D. Eberhagen, «Untersuchung und Bestimmung der Lipide im Blut», Springer-Verlag, Berlin, Heidelberg, New York, 1965) angegebenen Verfahren bestimmt (s = ± 2%). Als Vergleichssubstanz diente Kaliumdihydrogenphosphat. Der Gehalt an Cholesterin wurde mit einem kolorimetrischen Verfahren bestimmt, bei den neben dem Cholesterin auch der Cholesterinester erfaßt wird (s = ± 4%). Die Neutrallipide ließen sich mit dem Triglycerid-Bestimmungsverfahren nach DAB 8 bzw. dem 2. Arzneibuch der DDR, Methode 1 A.1.1., erfassen. Der Proteingehalt wurde nach einem von Hesse und Lindner (Zeitschrift für Allg. Mikrobiologie 11 (1971) 585–594; loc. cit. 15 (1975) 559–561) beschriebenen Verfahren bestimmt, das sich besonders gut zur Ermittlung von Proteinkonzentrationen in wäßrigen Emulsionen eignet (s = ± 3%). Die Bestimmung der einzelnen Phospholipide wurde nach dünnschichtchromatographischer Trennung nach P. Genter et al. (J. of Chromatography 206 (1981) 200–204) kolorimetrisch (siehe N. Zöllner und D. Eberhagen, «Untersuchung und Bestimmung der Lipide im Blut»; Springer-Verlag, Berlin, Heidelberg, New York, 1965) durchgeführt (s = ± 3% für die Hauptkomponenten). Als Vergleichssubstanz diente Kaliumhydrogenphosphat. Der Elutionsverlust der Phospholipide liegt innerhalb der Streuung. Die Ermittlung des Gehalts an freien Fettsäuren erfolgt nach dünnschichtchromatographischer Trennung und Elution im Gaschromatographen (s = ± 10%).

Durch das erfindungsgemäße Präparat wird die Oberflächenspannung einer wäßrigen Phase verändert. Die Messung der Oberflächenspannung und die Aufnahme der Hysteresekurven erfolgten mit einer Wilhelmy-Waage Typ ATF 01 (Hersteller Fa. E. Biegler, 301 Mauerbach, Österreich) nach bekanntem Verfahren.

Eine weitere Verbesserung der Stabilität und Emulgierbarkeit der Präparationen ist durch Zusatz einer Phospholipidfraktion erzielbar, die wie folgt gewonnen wird:

Nach Lösen des getrockneten erfindungsgemäßen Lungen-Surfactant-Präparats der oben angeführten Zusammensetzung in einem Lipidlösungsmittel, vorzugsweise Chloroform, in einer Konzentration von 0,1 bis 1,0 g/ml Lipidlösungsmittel wird eine Acetonfällung der Phospholipidfraktion bei −20 bis +20°C und vorzugsweise bei +5°C vorgenommen. Das Volumverhältnis von Wirkstofflösung zu Aceton liegt zwischen 1:1 und 1:8 und beträgt vorzugsweise 1:4. Nach Abtrennung der Mutterlauge durch Filtration oder Zentrifugieren und Waschen der festen Phase mit Aceton, das vorzugsweise auf −10°C bis −20°C temperiert ist, wird die Phospholipidfraktion getrocknet. Die so erhaltene nahezu weiße Substanz, die zu 70 bis 90% aus Phospholipiden besteht und keine biologische Aktivität zeigt, kann dem durch Alkoholextraktion gewonnenen Surfactant zugesetzt werden.

Das so erhaltene getrocknete Gemisch besitzt folgende Zusammensetzung (bestimmt nach den oben angegebenen Verfahren):

| | (%) |
|---|---|
| Phospholipide | 60–75 |
| Neutrallipide | 5–15 |
| Cholesterin | 10–20 |
| Protein | 0,5–1,5 |
| freie Fettsäuren | 2–5. |

Die Phospholipide liegen dabei in folgenden Mengenverhältnissen vor:

| | (%) |
|---|---|
| Phospholipidcholin | 60–70 |
| Phosphatidylglycerin | 2–4 |
| Phosphatidylethanolamin | 5–10 |
| Phosphatidylserin | 1–3 |
| Sphingomyelin | 10–20 |
| Phosphatidylinosit | 2–3. |

Nach diesem Verfahren gewonnene Präparate zeigten bei In-vivo-Tests im Tierversuch eine hohe Wirksamkeit und waren bei intratrachealer Instillation ausnahmslos gut verträglich, wie aus den Ergebnissen des experimentellen Teils hervorgeht.

Auch die durch wiederholte Alkoholextraktion und Acetonfällung gewonnene Phospholipidfraktion kann in analoger Weise verwendet werden.

Herstellung eines pharmazeutischen Mittels

Aus dem erhaltenen, dem natürlichen entsprechenden Surfactant mit einem Phospholipidgehalt zwischen 40 und 70% wurde nach folgendem Verfahren ein pharmazeutisches Mittel hergestellt:

Die Substanz wird nach Lösen in Ethanol oder halogenierten Kohlenwasserstoffen über Glasfilter und Filterpapier vorgeklärt und anschließend über lösungsmittelbeständige Membranen sterilfiltriert. Nach Abdampfen des Lösungsmittels im Vakuum wird der erhaltene Rückstand mit Wasser zur Injektion oder sterilen wäßrigen Lösungen emulgiert, in Glasgefäße abgefüllt, tiefgefroren und lyophilisiert.

Als wäßrige Lösungen können dabei isotonische Natriumchlorid- oder Calciumchloridlösungen bzw. isotonische Lösungen von Mono- und Disacchariden eingesetzt werden. Zum Homogenisieren eignet sich vorteilhaft ein Ultra-Turrax-Rührgerät. Außerdem ist ein Zusatz von geeigneten Mitteln zur Verbesserung der Emulgierfähigkeit und zur Erhöhung der Stabilität sowie von Puffern zur Einstellung bestimmter pH-Bereiche möglich.

Eine andere Möglichkeit zur Herstellung pharmazeutischer Mittel besteht darin, das erhaltene Surfactant unter Umgehung der Sterilfiltration in organischen Lösungsmitteln gleich in Wasser oder den oben genannten wäßrigen Lösungen zu emulgieren, wobei die oben aufgeführten Zusätze hier ebenfalls verwendbar sind, und die Emulsion zu autoklavieren. Nach Abkühlung kann erforderlichenfalls nochmals emulgiert werden. Anschließend wird die Emulsion in Glasgefäße abgefüllt, tiefgefroren und lyophilisiert.

Die folgenden Ausführungsbeispiele erläutern die Erfindung.

Beispiel 1

4,1 kg frische Rinderlunge wurden von Fettgewebe, größeren Tracheateilen sowie anhaftendem Blut befreit und zerteilt. Anschließend wurden die Lungenstücke in einem Fleischwolf mit 4-mm-Lochscheibe zerkleinert. Der so erhaltene Brei aus Lungengewebe (3,5 kg) wurde mit 14,0 l isotonischer Natriumchloridlösung durch einstündiges intensives Rühren bei 20 °C extrahiert.

Nach Absaugen über Dederongaze konnten 12,4 l eines trüben Extrakts gewonnen werden, der 1 h lang bei 20 °C mit 7,6 l Chloroform ausgerührt wurde. Nach 3 Tagen Stehenlassen wurde der entstandene feine Proteinniederschlag während 15 min bei einer relativen Zentrifugalbeschleunigung (RZB-Wert) von 2000 abzentrifugiert. Man erhielt 5,81 l einer klaren Chloroformphase, die nach Aufkonzentrieren im Rotationsverdampfer unter Stickstoff zur Trockne eingeengt wurde. Es wurden 6,14 g Substanz erhalten.

### Beispiel 2

200 kg Lungengewebe wurden von Fettgewebe und großen Tracheateilen befreit und in einem Fleischwolf mit 8-mm-Lochscheibe zerkleinert. Der Brei aus Lungengewebe wurde mit 600 l isotonischer Natriumchloridlösung in einem Industrieextraktor 30 min bei 17 °C gerührt. Nach 15 min Standzeit sammelte sich der überwiegende Teil des Lungengewebes über der wäßrigen Phase. Diese konnte vom Boden des Extraktors abgezogen und über einen Separator von noch enthaltenen Lungenteilen und Blutzellen befreit werden. Die erhaltenen 410 l wäßriger Extrakt wurden in 200-l-Kippkesseln weiterverarbeitet. In zwei Arbeitsgängen wurden je 130 l Extrakt mit jeweils 1,1 kg wasserfreiem Calciumchlorid versetzt und mit je 32,0 l Chloroform (DAB 8) 25 min bei 15 °C gerührt; ferner wurden 150 l Extrakt mit 1,2 kg wasserfreiem Calciumchlorid und 36,0 l Chloroform (DAB 8) versetzt und 25 min gerührt.

Nach Stehenlassen über Nacht wurde jeweils die wäßrige Oberphase und damit ein Teil der gefällten Proteine abdekantiert und der Rest über eine Schälzentrifuge abgetrennt. Es wurden etwa 60 l Chloroformphase erhalten, die im Vakuumumlaufverdampfer und anschließend im Rotationsumlaufverdampfer eingeengt wurden. Die Lösungsmittelreste wurden mit Stickstoff ausgetrieben. Es wurden 513 g Substanz erhalten.

### Beispiel 3

2,0 kg wie in Beispiel 1 aus 2,2 kg Rinderlunge gewonnener Brei aus Lungengewebe wurden in 5,0 l Chloroform 60 min bei 20 °C gerührt. Nach dem Abfiltrieren des Lungengewebes wurde der Chloroformextrakt nach Aufkonzentrierung im Rotationsverdampfer unter Stickstoff zur Trockne eingeengt. Es wurden 2,84 g Substanz erhalten.

### Beispiel 4

276 kg frische Rinderlunge wurden von Fettgewebe und größeren Tracheateilen befreit und in einem Industriefleischwolf mit 8-mm-Lochscheibe zerkleinert. Die so erhaltenen 250 kg Brei aus Lungengewebe wurden mit 300 l Chloroform bei 22 °C in einem Industrieextraktor 60 min ausgerührt. Die Chloroformphase wurde nach 50 min Stehenlassen abgelassen, im Vakuumumlaufverdampfer und im Vakuumrotationsverdampfer aufkonzentriert und durch Stickstoffeinleitung zur Trockne eingeengt. Es wurden 950 g Substanz erhalten.

### Beispiel 5

7,2 kg frische Rinderlunge wurden von Fettgewebe und größeren Tracheateilen befreit und nach Abwaschen von anhaftendem Blut in einem Fleischwolf zerkleinert. Die erhaltenen 6,0 kg Brei aus Lungengewebe wurden portionsweise mit isotonischer Natriumchloridlösung 30 min ausgerührt, wobei auf 2,5 kg Lungengewebe 8,0 l wäßrige Lösung eingesetzt wurden. Nach Abtrennung der Rückstände des Lungengewebes über Dederongaze wurden 8,8 l der erhaltenen 17,6 l Extrakt mit 59,0 g wasserfreiem Calciumchlorid versetzt und mit 2,2 l Tetrachlorkohlenstoff 30 min ausgerührt, wobei 1,97 l organische Phase erhalten wurden. Nach Abdampfen des Lösungsmittels wurden 2,34 g Substanz erhalten.

### Beispiel 6

Frische Rinderlunge wurde von Fettgewebe, größeren Tracheateilen sowie anhaftendem Blut befreit und dabei zerteilt. Anschließend wurden die Lungenstücke in einem Fleischwolf mit 4-mm-Lochscheibe zerkleinert. Die so erhaltenen 65,0 kg Brei aus Lungengewebe wurden in 5 Portionen von je 13,0 kg mit je 15,6 l Chloroform 30 min bei Raumtemperatur ausgerührt. Nach dem Abfiltrieren des Lungengewebes wurden zwischen 10,5 und 11,5 l Chloroformextrakt erhalten. Die 5 Extrakte wurden vereinigt, im Rotationsverdampfer aufkonzentriert und unter Stickstoff zur Trockne eingeengt. Es wurden 254 g rohes Surfactant erhalten. 55 g davon wurden mit 105 ml Ethanol (96%) bei Raumtemperatur mittels einem Rührwerk (Ultra-Turrax) 5 min ausgerührt. Die Temperatur stieg dabei auf 62 °C an. Der unlösliche Rückstand wurde abfiltriert und der klare Ethanolextrakt im Rotationsverdampfer aufkonzentriert; danach wurde das Lösungsmittel unter Stickstoff völlig ausgetrieben. Es wurden 11,9 g reines Surfactant erhalten.

### Beispiel 7

Aus 12,0 kg Brei aus Lungengewebe, der nach Beispiel 1 hergestellt wurde, konnten nach 30 min Ausrühren mit 15,0 l Chloroform bei Raumtemperatur 10,2 l Extrakt gewonnen werden. Nach Abdampfen des Lösungsmittels wurden 50,24 g rohes Surfactant erhalten.

20 g dieses rohen Surfactants wurden mit 80 ml Ethanol 5 min bei −10 °C ausgerührt. Nach Aufarbeitung wie in Beispiel 6 wurden 3,89 g reines Surfactant erhalten.

### Beispiel 8

Aus 16,0 kg Brei aus Lungengewebe, der nach Beispiel 6 hergestellt wurde, konnten nach 30 min Ausrühren mit 18,2 l Chloroform bei Raumtemperatur 14 l Extrakt gewonnen werden. Nach Abdampfen des Lösungsmittels blieben 63,1 g rohes Surfactant zurück. 26 g dieses rohen Surfactants wurden mit 80 ml Ethanol 5 min bei 15 bis 20 °C ausgerührt. Nach Aufarbeitung wie in Beispiel 6 wurden 8,45 g reines Surfactant erhalten.

Beispiel 9

Aus 125,0 kg Brei aus Lungengewebe, der nach Beispiel 6 hergestellt wurde, konnten nach portionsweisem Ausrühren mit insgesamt 150 l Chloroform bei Raumtemperatur während jeweils 30 min 102,6 l Extrakt gewonnen werden. Nach Abdampfen des Lösungsmittels wurden 520 g rohes Surfactant erhalten.

500 g dieses rohen Surfactants wurden mit 1500 ml Ethanol 15 min bei 10 bis 20 °C ausgerührt. Der erhaltene Extrakt wurde wie in Beispiel 6 aufgearbeitet. Es wurden 198 g reines Surfactant erhalten.

Beispiel 10

Aus 150 g reinem Surfactant von Beispiel 9 wurde nach Auflösen in 320 ml Chloroform und Abkühlen auf 0 °C durch Zusatz von 960 ml tiefgekühltem Aceton (−20 °C) eine Phospholipidfraktion ausgefällt. Die Fällung wurde nach 3 h Stehenlassen abfiltriert, ausgefällt und getrocknet. Es wurden 97,0 g Phospholipidfraktion erhalten.

Beispiel 11

39,4 g rohes Surfactant von Beispiel 8 wurden mit 117,6 ml Methanol 15 min bei 28 bis 34 °C ausgerührt. Nach Aufarbeitung gemäß Beispiel 6 wurden 13,83 g reines Surfactant erhalten.

Beispiel 12

Aus 196,0 kg Brei aus Lungengewebe wurden nach dem Verfahren von Beispiel 6 723 g rohes Surfactant gewonnen. 603 g rohes Surfactant wurden mit 1809 ml Ethanol 15 min bei 15 bis 20 °C ausgerührt. Aus dem ethanolischen Extrakt wurden nach Abdampfen des Lösungsmittels 240 g reines Surfactant erhalten. Dabei blieben 365 g in Ethanol unlöslicher Rückstand zurück.

Beispiel 13

Die 365 g Rückstand von Beispiel 12 wurden erneut mit 1200 ml Ethanol unter den gleichen Bedingungen ausgerührt. Aus dem Extrakt wurden nochmals 59 g reines Surfactant erhalten. Dabei blieben 314 g in Ethanol unlöslicher Rückstand zurück.

Beispiel 14

40 g reines Surfactant von Beispiel 13 wurden in Chloroform zu insgesamt 100 ml gelöst. Nach Abkühlen dieser Lösung auf 8 °C wurde die Phospholipidfraktion durch Zusatz von 300 ml tiefgekühltem Aceton ausgefällt. Der Niederschlag wurde abgesaugt und getrocknet. Es wurden 16,9 g Phospholipidfraktion erhalten.

Beispiel 15

4,5 kg frische Rinderlunge wurden von anhaftendem Fettgewebe, größeren Blutgefäßen und Tracheateilen befreit und zerteilt. Nach Waschen der Lungenstücke, wobei diese vor allem von anhaftendem Blut befreit wurden, erfolgte die Zerkleinerung des Lungengewebes in einem Fleischwolf mit 4-mm-Lochscheibe. Der so erhaltene Brei aus Lungengewebe wurde in Portionen zu 500 g mit je 2,0 l Saccharoselösung (114 g/l) 10 min lang intensiv geschüttelt. Durch Filtrieren über ein Kunststoffsieb und anschließendes Zentrifugieren des Filtrats bei einem RZB-Wert von 500 und 20 °C wurden 15,4 l wäßriger Extrakt erhalten.

7,0 l dieses Extrakts wurden mit 420 g Kieselgel für die Säulenchromatographie 60 min bei 20 °C gerührt. Nach 16 h Stehenlassen bei 4 °C wurde der klare Überstand abgehebert und verworfen. Das Kieselgel mit adsorbiertem Surfactant wurde nach Absaugen des restlichen Überstands mit 1 l destilliertem Wasser auf einer G 2-Fritte gewaschen. Die Elution des Surfactants erfolgte mit 1,5 l Chloroform-Methanol-Gemisch (2:1). Die Chloroformphase wurde abgetrennt und unter Stickstoff zur Trockne eingeengt. Im Ergebnis wurden 515 mg Substanz erhalten.

Beispiel 16

8,4 l des in Beispiel 15 beschriebenen wäßrigen Extrakts wurden mit 500 g Florisil® 30 min bei 20 °C gerührt. Nach 16-h-Stehenlassen bei 4 °C wurde der klare Überstand abgehebert und verworfen. Die weitere Aufarbeitung erfolgte wie in Beispiel 15. Es wurden 600 mg Substanz erhalten.

Beispiel 17

2,5 kg Brei aus Lungengewebe, der wie in Beispiel 15 hergestellt war, wurde lyophilisiert und nach 3-wöchiger Kühllagerung, wie ebenfalls in Beispiel 15 beschrieben, zu einem wäßrigen Extrakt aufgearbeitet. Dabei wurde das Lungenlyophilisat entsprechend seinem ursprünglichen Frischgewicht eingesetzt. Als Extraktionsmittel diente in Abweichung von Beispiel 15 Glucoselösung (55 g/l).

7,1 l des wäßrigen Extrakts wurden mit 420 g Kieselgel für die Säulenchromatographie 60 min bei 20 °C gerührt. Nach Aufarbeitung des Adsorbats nach der in Beispiel 15 erläuterten Variante wurden 310 mg Substanz erhalten.

Beispiel 18

36,0 kg frische Rinderlunge wurden wie in Beispiel 1 zu einem Brei aus Lungengewebe verarbeitet. Je 500 g dieses Breis wurden jeweils mit 2,0 l isotonischer Natriumchloridlösung 60 min bei 20 °C intensiv gerührt. Nach Filtrieren über Dederongaze und anschließendes Zentrifugieren des Filtrats bei einem RZB-Wert von 500 und 20 °C konnten 120 l wäßriger Extrakt gewonnen werden.

Jeweils 30 l Extrakt wurden mit 2,5 kg Florisil® 60 min bei 20 °C gerührt. Nach dem Absetzen des Adsorptionsmittels wurde der Überstand (jeweils 26 bis 28 l) abdekantiert und erneut mit 2,0 kg Florisil® 60 min bei 20 °C gerührt. Die einzelnen Florisiladsorbate wurden dann mit je 5 l destilliertem Wasser gewaschen und mit je 6,4 l Chloroform-Methanol-Gemisch (2:1) eluiert.

Die Chloroformphasen wurden vereinigt. Nach Einengen unter Stickstoff wurden 12,2 g Substanz erhalten.

## Beispiel 19

Aus 9,35 kg frischer Rinderlunge wurden nach Beispiel 15 in 500-g-Portionen, die mit je 2,0 l isotonischer Natriumchloridlösung bei 20 °C intensiv gerührt wurden, 31,7 l wäßriger Extrakt gewonnen. 23,0 l dieses Extrakts wurden mit 1,5 kg Florisil® 60 min bei 20 °C gerührt. Nach Abtrennen des Überstands wurde das Surfactant-beladene Florisil® in zwei gleiche Teile geteilt. Der eine Teil wurde mit 4 l Leitungswasser, der andere mit 3 l Aceton gewaschen. Jeder Teil wurde anschließend mit 2 l Chloroform-Methanol-Gemisch (2:1) eluiert; die Chloroformphasen wurden abgetrennt und unter Stickstoff zur Trockne eingedampft. Das mit Wasser gewaschene Absorbat lieferte 1,0 g, das mit Aceton gewaschene Absorbat 0,62 g Substanz.

## Beispiel 20

8,0 l des nach Beispiel 19 gewonnenen Extrakts wurden mit 1,2 kg Kieselgel 60 min bei 20 °C gerührt. Nach Abtrennen des Überstands wurde das Surfactant-beladene Kieselgel mit 4 l Leitungswasser gewaschen und mit 2 l Dichlormethan-Methanol-Gemisch (2:1) eluiert. Die zur Trockne eingedampfte Dichlormethanphase ergab 422 mg Substanz.

## Beispiel 21

Aus 9,0 l wäßrigem Extrakt, der wie in Beispiel 19 hergestellt war, konnten nach Ausrühren mit 2 kg Kieselgur, Waschen des Absorbats mit 10 l Leitungswasser und Elution des Surfactant-Materials durch 60 min Rühren in 2,8 l Chloroform-Methanol-Gemisch (2:1) 600 ml Chloroformphase erhalten werden. Diese wurde unter Stickstoff zur Trockne eingedampft und ergab 1,3 g Substanz.

## Beispiel 22

Aus 3,0 l wäßrigem Extrakt, der wie in Beispiel 19 hergestellt worden war, konnten nach Ausrühren mit 0,68 kg Kieselgel, das bereits in Beispiel 19 verwendet worden war und durch Behandlung mit 2 N NaOH regeneriert werden konnte, Waschen des Adsorbats mit 2 l Aceton und Elution des Surfactant-Materials durch 60 min Rühren in 1000 ml Chloroform-Methanol-Gemisch 960 ml Chloroformphase erhalten werden. Diese wurde unter Vakuum zur Trockne eingedampft und ergab 0,6 g Substanz.

## Beispiel 23

9,0 kg Brei aus Lungengewebe, der wie in Beispiel 1 hergestellt worden war, wurden in 18 Portionen zu 0,5 kg mit je 2,0 l isotonischer Kochsalzlösung durch 10 min kräftiges Schütteln extrahiert, worauf die Rückstände des Lungengewebes über ein Kunststoffsieb und Dederongaze abgetrennt wurden. Die erhaltenen 34 l Extrakt wurden auf eine mit 2,0 kg Florisil® gefüllte Säule (Durchmesser 15 cm, Füllhöhe 25 cm) aufgegeben. Anschließend wurde mit 10,0 l destilliertem Wasser gewaschen und der Wirkstoff mit 7,5 l Chloroform-Methanol-Gemisch (2:1) eluiert. Nach Stehenlassen über Nacht konnte die Methanolphase des Eluats abgesaugt werden. Die Chloroformphase wurde im Vakuum zur Trockne eingeengt. Es wurden 2,65 g Surfactant erhalten.

## Beispiel 24

20 g erfindungsgemäßer Wirkstoff (Phospholipidgehalt 60,1%) wurden in 200 ml unvergälltem Ethanol gelöst, über eine G 4-Fritte (aufgelegtes Glasfaserfilterpapier NK VIII) vorfiltriert und anschließend sterilfiltriert (über Sartorius-Filter SM 116-07). Nach Aufkonzentrieren der sterilen Lösung im Vakuumrotationsverdampfer wurden durch Einleitung von sterilem Stickstoff bei gleichzeitiger Evakuierung des Gefäßes die Lösungsmittelreste abgedampft. Die so erhaltene sterile Substanz wurde mit 180 ml sterilem Wasser mit einem Rührwerk (Ultra-Turrax) emulgiert. Die Emulsion wurde so in 25-ml-Rollrandflaschen abgefüllt, daß 17 Flaschen mit je 400 mg Phospholipid erhalten wurden. Nach Einfrieren bei −50 °C für 3 Tage wurden die Proben lyophilisiert, wobei die Gegenheizung auf maximal 20 °C eingestellt wurde. Nach der Gefriertrocknung wurden die Flaschen mit Durchstechstopfen (B 180) und Aluminium-Kapselverschlüssen verschlossen.

## Beispiel 25

12 g reines Surfactant von Beispiel 9 und 12 g der Phospholipidfraktion von Beispiel 10 wurden gemeinsam emulgiert und wie in Beispiel 24 zu einem pharmazeutischen Mittel verarbeitet.

## Beispiel 26

5,0 g erfindungsgemäßer Wirkstoff (Phospholipidgehalt 56,8%) wurden in 50,0 ml Chloroform (DAB 8) gelöst, über Glasfaserfilterpapier (Schichtdicke 1,80 mm) und Filtrierpapier vorfiltriert sowie anschließend über Glasfaservorfilter (Schichtdicke 450 µm) auf Cellulose-Sterilfiltern (0,2 µm Porendurchmesser, 90 µm Schichtdicke) sterilfiltriert. Nach Aufkonzentrieren der sterilen Lösung im Vakuumrotationsverdampfer wurden durch Einleiten von sterilem Stickstoff bei gleichzeitiger Evakuierung des Gefäßes die Lösungsmittelreste abgedampft. Die so erhaltene sterile Substanz wurde mit 40,0 ml sterilem Wasser mit einem Rührwerk (Ultra-Turrax) emulgiert. Die erhaltene Emulsion wurde so in 25-ml-Rollrandflaschen abgefüllt, daß 7 Flaschen mit je 400 mg Phospholipid erhalten wurden. Nach Einfrieren bei −50 °C für 3 Tage wurden die Proben lyophilisiert, wobei die Gegenheizung auf maximal 20 °C eingestellt wurde. Nach der Gefriertrocknung wurden die Flaschen mit Durchstechstopfen S 180 und Aluminium-Kapselverschlüssen verschlossen.

Die Eigenschaften der in den Beispielen 1–18 hergestellten Präparate sind in Tabelle 1 zusammengefaßt.

Die grenzflächenaktive Wirksamkeit der Präparationen der Beispiele 2, 3, 9, 10 und 18 wurde anhand der Oberflächenspannung einer wäßrigen Phase nach dem oben angegebenen Verfahren bestimmt.

Substanzeinsatz: 5 µl mit 60 mg Phospholipid/ml.

Tabelle 1    Chemische Zusammensetzung (%)

| | Gesamt-PL | PC | PE | PG | Sph | PS | PI | Protein | Neutral-lipide | Cholesterin | freie Fettsäuren | Wasser |
|---|---|---|---|---|---|---|---|---|---|---|---|---|
| Beispiel 1 | 57,5 | nicht bestimmt | nicht bestimmt | | | | | 0,32 | | nicht bestimmt | nicht bestimmt | |
| Beispiel 2 | 30,0 | 16,4 | 5,2 | 0,5 | 3,5 | 0,2 | 0,5 | 0,83 | 61,3 | 9,9 | 3,6 | 1,3 |
| Beispiel 3 | 40,0 | nicht bestimmt | nicht bestimmt | | | | | 1,5 | | nicht bestimmt | nicht bestimmt | |
| Beispiel 4 | 26,4 | 13,3 | 3,5 | 0,3 | 3,6 | 0,3 | 0,4 | 1,2 | 54,2 | 13,5 | 2,1 | 2,4 |
| Beispiel 5 | 53,2 | nicht bestimmt | nicht bestimmt | | | | | 0,4 | | nicht bestimmt | nicht bestimmt | |
| Beispiel 6 | 62,4 | 41,2 | 8,1 | 0,7 | 7,5 | 0,2 | 0,8 | 0,6 | 17,3 | 14,2 | 2,4 | |
| Beispiel 7 | 55,6 | 34,6 | 7,9 | 1,5 | 6,1 | 1,2 | 0,5 | 0,8 | 13,3 | 14,6 | 2,4 | |
| Beispiel 8 | 58,5 | 39,4 | 9,5 | 1,0 | 5,7 | 0,2 | 0,6 | n.b. | 10,8 | 15,1 | 2,3 | |
| Beispiel 9 | 59,8 | 38,3 | 7,5 | 0,0 | 8,9 | 0,8 | 0,5 | 1,5 | 15,9 | 16,0 | 3,3 | |
| Beispiel 10') | 74,7 | 48,4 | 9,7 | 0,0 | 12,5 | 0,7 | 0,5 | 1,5 | 2,2 | 9,1 | 2,5 | |
| Beispiel 10 | 64,4 | 40,5 | 8,7 | 0,0 | 9,9 | 0,4 | 0,3 | 1,3 | 8,1 | 12,4 | 2,8 | |
| Beispiel 11 | 67,0 | 34,0 | 3,6 | 0,9 | 11,3 | 3,8 | 1,3 | n.b. | 4,2 | 15,5 | 4,6 | |
| Beispiel 12 | 60,2 | 37,5 | 8,9 | 1,1 | 7,3 | 1,7 | 0,5 | 0,9 | 12,5 | 16,7 | 2,7 | |
| Beispiel 13 | 53,4 | 30,1 | 11,3 | 1,0 | 7,5 | 0,6 | 0,4 | n.b. | 17,0 | 23,8 | n.b.'') | |
| Beispiel 14') | 77,6 | 43,7 | 15,5 | 1,3 | 12,9 | 1,5 | 0,5 | 1,2 | 3,3 | 10,4 | n.b. | |
| Beispiel 15 | 23,0 | nicht bestimmt | nicht bestimmt | | | | | 0,3 | 28,2 | 41,2 | n.b. | |
| Beispiel 16 | 40,0 | nicht bestimmt | nicht bestimmt | | | | | 0,6 | 23,4 | 30,1 | n.b. | |
| Beispiel 17 | 21,5 | nicht bestimmt | nicht bestimmt | | | | | 0,4 | 31,3 | 38,3 | n.b. | |
| Beispiel 18 | 25,0 | nicht bestimmt | nicht bestimmt | | | | | 0,2 | 19,5 | 39,4 | n.b. | |
| Beispiel 19 | | | | | | | | | | | | |
| Variante mit Wasser | 34,2 | nicht bestimmt | nicht bestimmt | | | | | 0,3 | 16,2 | 40,2 | nicht bestimmt | |
| Variante mit Aceton | 40,1 | nicht bestimmt | nicht bestimmt | | | | | 0,1 | 12,3 | 37,3 | nicht bestimmt | |
| Beispiel 20 | 21,2 | nicht bestimmt | nicht bestimmt | | | | | 0,3 | 28,7 | 39,3 | nicht bestimmt | |
| Beispiel 21 | 40,0 | nicht bestimmt | nicht bestimmt | | | | | 0,4 | 17,4 | 31,6 | nicht bestimmt | |
| Beispiel 22 | 50,0 | nicht bestimmt | nicht bestimmt | | | | | 0,4 | 11,8 | 29,4 | nicht bestimmt | |
| Beispiel 23 | 38,2 | nicht bestimmt | nicht bestimmt | | | | | 0,5 | 18,3 | 33,7 | nicht bestimmt | |

') Phospholipidfraktion
'') nicht bestimmt

Abkürzungen:
PL  Phospholipid
PC  Phosphatidylcholin
PE  Phosphatidylethanolamin
PG  Phosphatidylglycerin
Sph  Sphingomyelin
PS  Phosphatidylserin
PI  Phosphatidylinosit

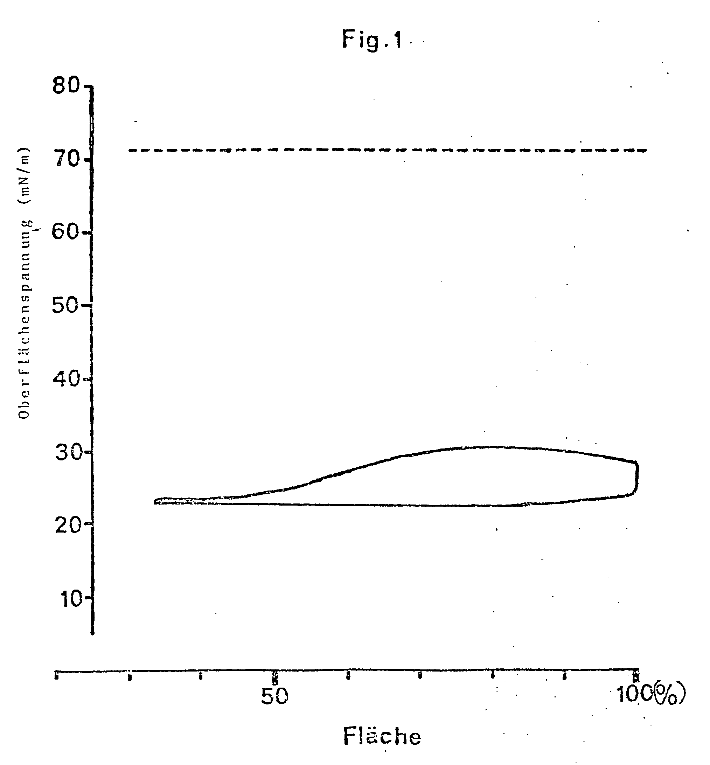

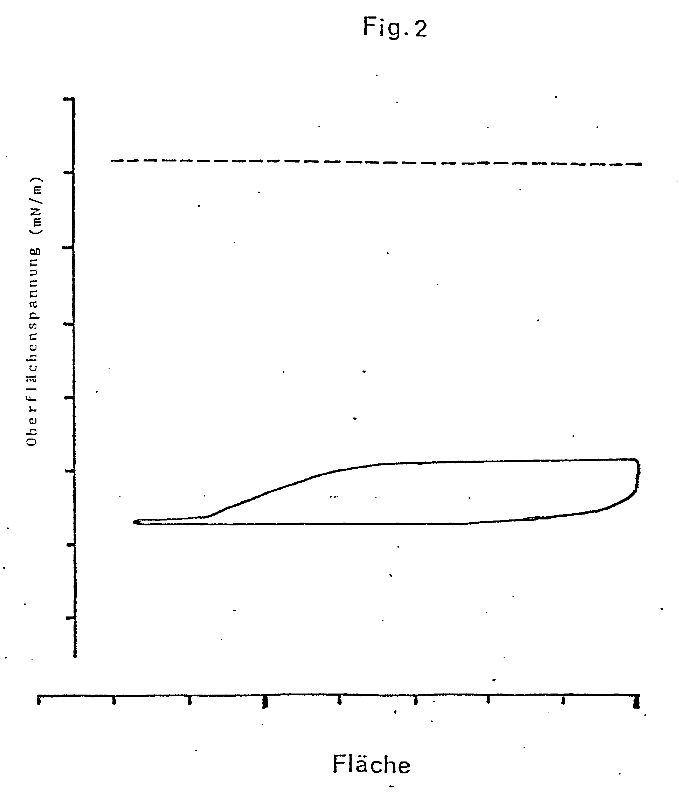

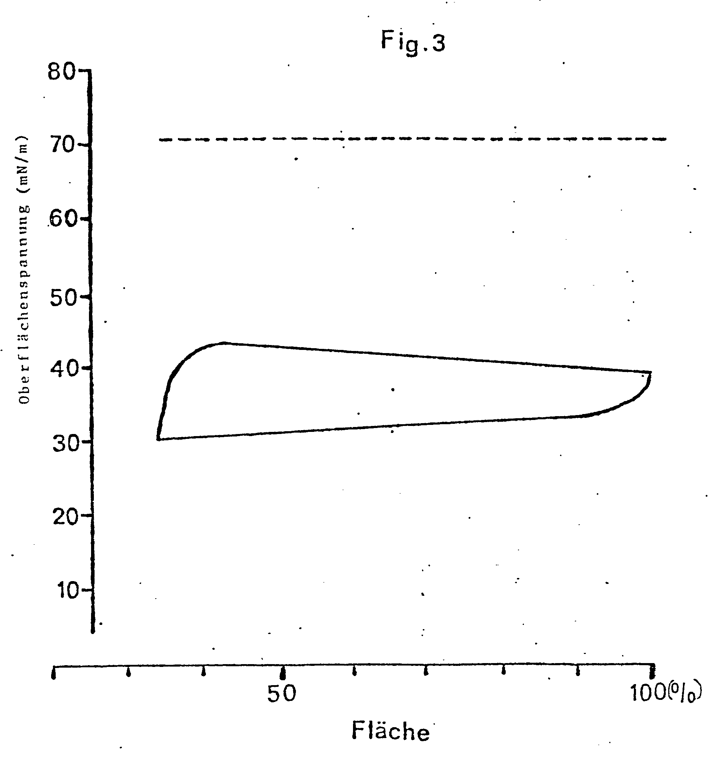

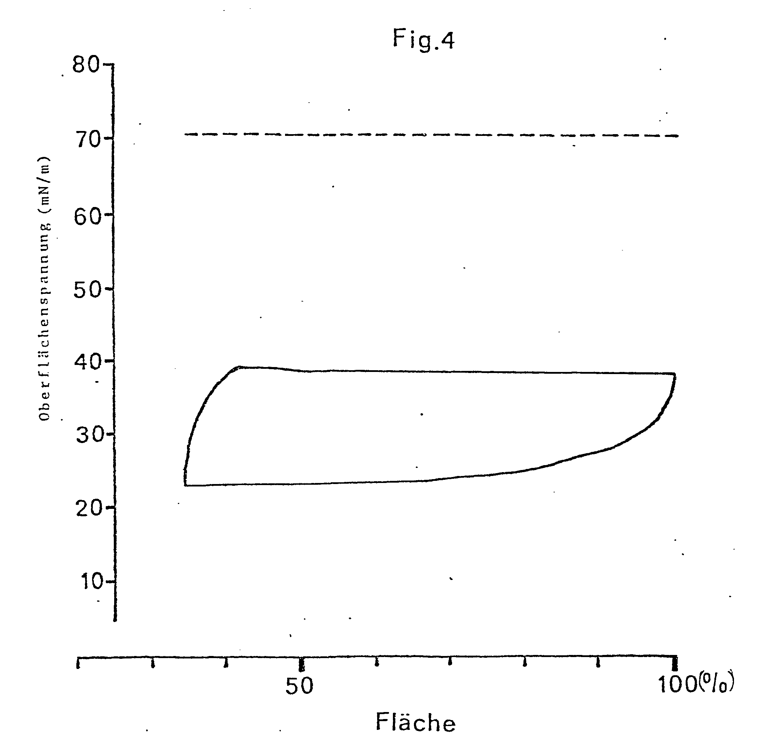

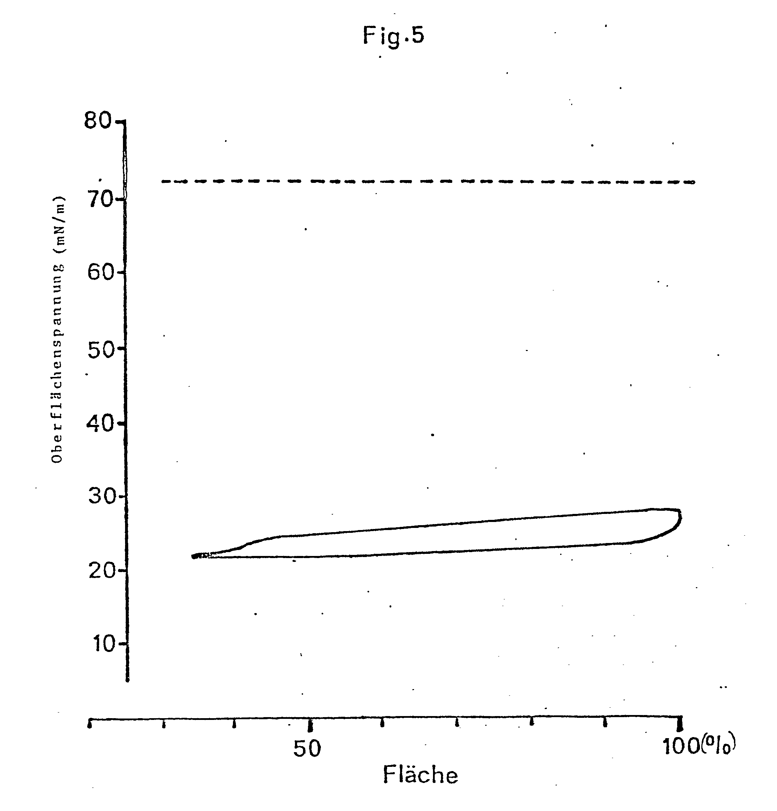

Die erhaltenen Ergebnisse der Oberflächenspannungsmessungen sind in den Fig. 1 bis 5 dargestellt:

Fig. 1: Substanz vom Beispiel 2
Fig. 2: Substanz vom Beispiel 3
Fig. 3: Substanz vom Beispiel 9
Fig. 4: Substanz vom Beispiel 10
Fig. 5: Substanz vom Beispiel 18

Als Abszisse ist dabei die relative Oberfläche angetragen.

Biologische Wirksamkeit und Verträglichkeit:

1. Einfluß von Surfactants auf unreife Lungen

Zur Untersuchung des Einflusses des Surfactants auf unreife Lungen wurden unreife Kaninchenneugeborene durch Hysterotomie am 27. Schwangerschaftstag geboren (Gesamtschwangerschaftsdauer 31 $\pm$ 1 Tag). Die Tiere wurden unmittelbar nach der Geburt tracheotomiert; ein Teil erhielt über eine Trachealkanüle durchschnittlich 60 µl Surfactant in die Trachea instilliert; unbehandelte Schwestertiere des gleichen Wurfes dienten als Kontrollen. Anschließend wurden die Tiere in einen druckkonstanten Ganzkörperplethysmographen (38 °C) gebracht und mit reinem Sauerstoff bei kontinuierlicher Registrierung von Beatmungsdruck und Atemvolumen beatmet. Aus den Primärdaten wurde die Thorax-Lungen-Compliance errechnet. Bei einigen Versuchen wurden alle Tiere nach der Geburt 10 bis 15 min beatmet, und erst dann erhielt ein Teil der Tiere Surfactant über die Trachea instilliert.

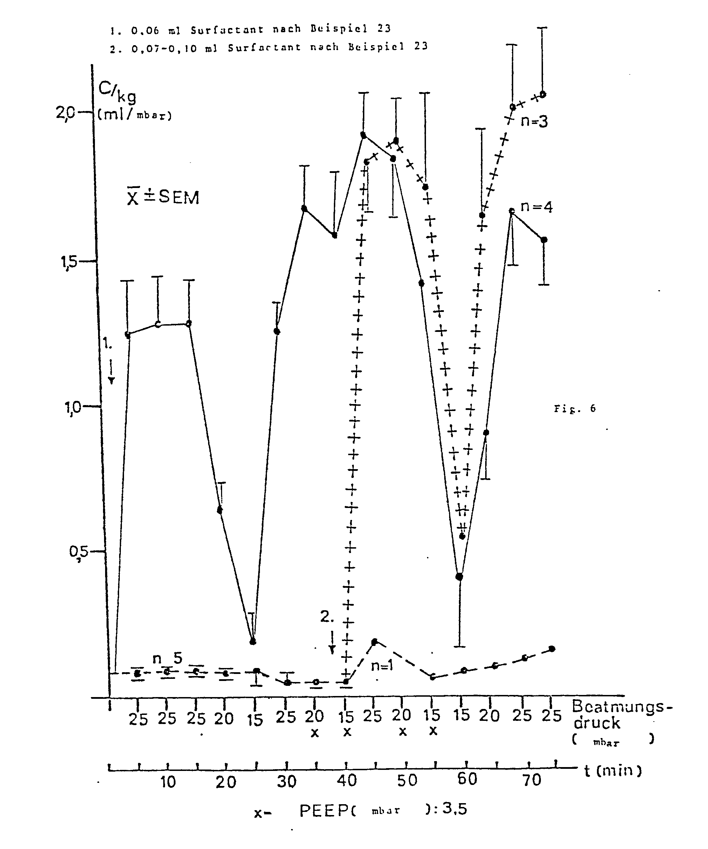

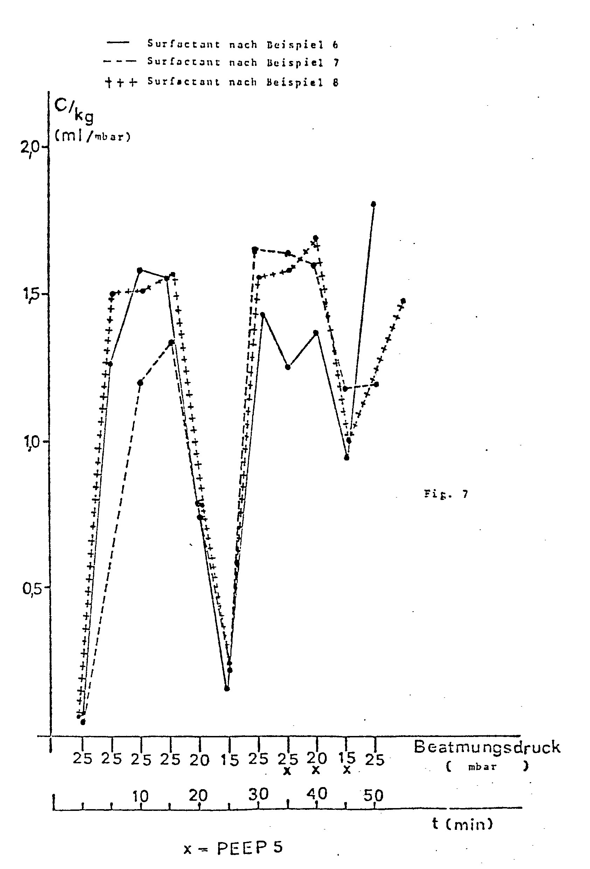

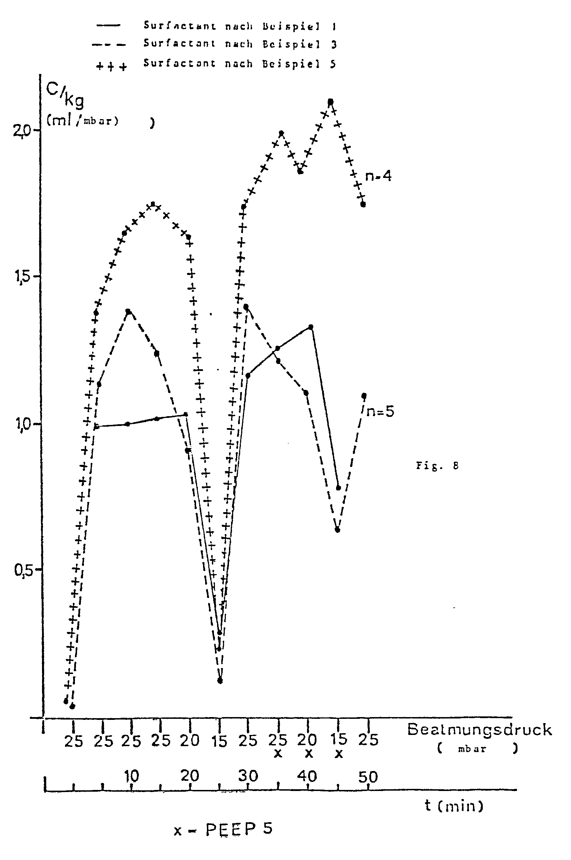

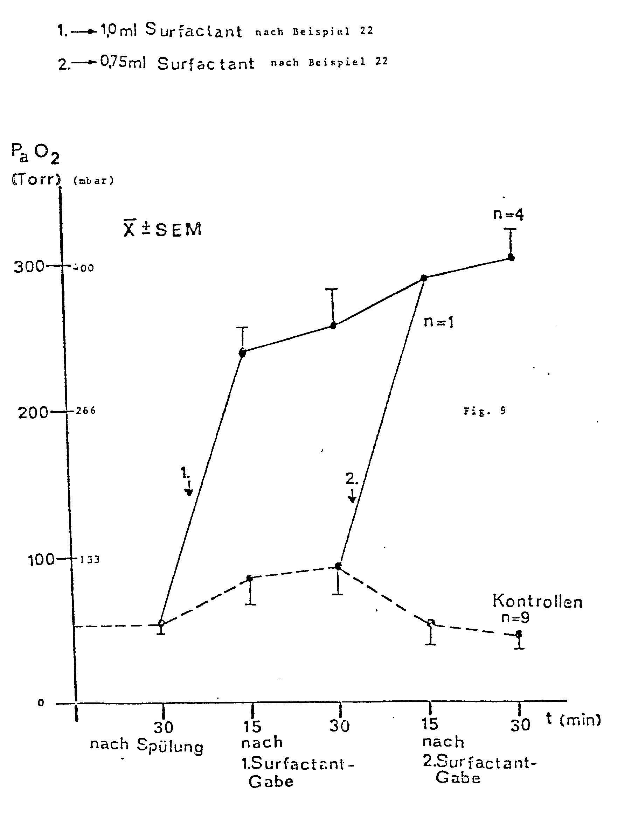

Die erhaltenen Ergebnisse sind in Tabelle 2 aufgeführt und in den Fig. 6 bis 8 dargestellt.

Nach dem erfindungsgemäßen Verfahren gewonnene Präparate zeigten bei In-vivo-Tests im Tierversuch eine hohe Wirksamkeit und waren bei intratrachealer Instillation ausnahmslos gut verträglich. Dabei wurde wie folgt verfahren:

2. Einfluß von Surfactants beim Erwachsenen-Atemnotsyndrom

Der Einfluß einer Surfactant-Instillation beim Erwachsenen-Atemnotsyndrom wurde an Meerschweinchen untersucht. Vor Erzeugung der akuten respiratorischen Insuffizienz wurden die Tiere nach allgemeiner Anaesthesie tracheotomiert; zur Blutentnahme wurde ein Katheter in die A. carotis eingeführt. Die Tiere wurden druckkonstant mit reinem Sauerstoff beatmet. Das akute Atemnotsyndrom wurde bei den Tieren durch 8-malige bronchiale Auswaschung des Surfactants mit warmer physiologischer Kochsalzlösung erreicht. Die Spülvolumina entsprachen der Vitalkapazität der Tiere. Am Ende der Spülprozedur lag der arterielle Sauerstoffdruck bei diesen Tieren, trotz Beatmung mit Spitzenüberdrucken von 30 mbar (30 cm $H_2O$), PEEP-Positive-Endexpiratory-Pressure)-Werten von 8 mbar (8 cm $H_2O$) und reinem Sauerstoff, unter 80 mbar (60 mm Hg). 10 und 40 min nach der letzten Lungenspülung erhielt die Hälfte der Tiere je 1 ml Surfactant tracheal instilliert. Die Blutgasanalysen erfolgten 5, 15 und 30 min nach der jeweiligen Surfactant-Verabreichung. Ein optimales Surfactant führt unter absolut gleichen Beatmungsbedingungen zu einer unmittelbaren Erhöhung des arteriellen Sauerstoffdrucks um durchschnittlich 266 mbar (200 mm Hg).

Die erhaltenen Ergebnisse sind in Tabelle 2 aufgeführt und in Fig. 9 dargestellt.

3. Prüfung auf Verträglichkeit nach trachealer Instillation an juvenilen Ratten

Die Prüfung auf Verträglichkeit an juvenilen Ratten erfolgte im Konzentrationsbereich der therapeutischen Dosis (200 mg Phospholipid/kg Körpermasse) bis zur dreifachen therapeutischen Dosis. Pro Ratte (ca. 50 g Körpermasse) wurden 10 bis 30 mg Phospholipid eingesetzt. Zu den Verträglichkeitstests wurden weibliche und männliche Tiere herangezogen, wobei bei einem Versuch (n = 10) nur Ratten gleichen Geschlechts ausgewählt wurden.

Methodik:

10 Ratten von ca. 50 g (männlich bzw. weiblich) wurden mit Ether narkotisiert; anschließend erfolgten Halsschnitt sowie stumpfe Präparation der Trachea. Nach der Präparation wurde die Ethernarkose vertieft, und zwar so, daß die Tiere weiterhin gleichmäßig durchatmeten. Mit einer gebogenen 20iger-Kanüle wurden jeweils 10 bis 30 mg Phospholipid in 0,3 ml physiologischer Kochsalzlösung injiziert. Nach erfolgter Injektion wurde der Schnitt vernäht. Der Beobachtungszeitraum betrug zwei Wochen, wobei nach 72 h der Endbefund festgestellt wurde. Pro Testserie (50 bis 60 Tiere) wurden fünf Kontrolltiere mitgeführt, denen jeweils 0,3 ml physiologische Kochsalzlösung injiziert wurden.

Die erhaltenen Ergebnisse sind in Tabelle 2 aufgeführt.

Tabelle 2
Biologische Wirksamkeit und Verträglichkeit

| | Verträg-lichkeit | Compliance/kg (ml/mbar) bei einem Beatmungsdruck von +25 mbar, 30 min nach Geburt | n | Arterieller Sauerstoffpartialdruck beim experimentellen Atemnotsyndrom durch Lungenspülung vor und nach Surfactant-Instillation (mbar) | | |
|---|---|---|---|---|---|---|
| | | | | vor Instilla-tion | 30 min nach Instillation | n |
| Beispiel 1 | n.b. | 1,18 ± 0,24 | 4 | | nicht bestimmt | |
| Beispiel 2 | n.b. | 1,01 ± 0,32 | 5 | | nicht bestimmt | |
| Beispiel 3 | n.b. | 1,36 ± 0,21 | 5 | 68 ± 11 | 298 ± 53 | 3 |
| Beispiel 4 | n.b. | 1,12 ± 0,24 | 8 | | nicht bestimmt | |
| Beispiel 5 | n.b. | 1,75 ± 0,31 | 4 | | nicht bestimmt | |
| Beispiel 6 | 1,0 | 1,4 ± 0,3 | 5 | 71 ± 5 | 486 ± 59 | 5 |
| Beispiel 7 | 0,9 | 1,6 ± 0,4 | 7 | 81 ± 11 | 408 ± 37 | 5 |
| Beispiel 8 | 1,0 | 1,6 ± 0,3 | 6 | | nicht bestimmt | |
| Beispiel 9 | 1,0 | 1,1 ± 0,4 | 4 | 64 ± 7 | 338 ± 97 | 6 |
| Beispiel 10[*) | 1,0 | 0,2 ± 0,1 | 5 | 72 ± 11 | 166 ± 51 | 4 |

Tabelle 2
(Fortsetzung)

| | Verträg-lichkeit | Compliance/kg (ml/mbar) bei einem Be-atmungsdruck von +25 mbar, 30 min nach Geburt | n | Arterieller Sauerstoffpartialdruck beim experimentellen Atemnotsyndrom durch Lungenspülung vor und nach Surfactant-Instillation (mbar) | | |
|---|---|---|---|---|---|---|
| | | | | vor Instilla-tion | 30 min nach Instillation | n |
| Beispiel 10 | 1,0 | 1,4±0,3 | 7 | 69±5 | 377±68 | 4 |
| Beispiel 11 | 0,9 | 1,3±0,2 | 6 | 75±13 | 414±81 | 5 |
| Beispiel 12 | 1,0 | 1,8±0,4 | 6 | 61±8 | 324±60 | 5 |
| Beispiel 13 | 1,0 | 1,6±0,4 | 7 | 95±20 | 301±51 | 4 |
| Beispiel 14[*] | 1,0 | 0,3±0,1 | 4 | | nicht bestimmt | |
| Beispiel 15 | n.b. | 1,01±0,73 | 4 | | nicht bestimmt | |
| Beispiel 16 | n.b. | 1,60±0,51 | 7 | 73±4 | 373±44 | 5 |
| Beispiel 17 | n.b. | 1,20±0,24 | 3 | 56±15 | 317±128 | 4 |
| Beispiel 18 | n.b. | 1,53±0,02 | 2 | 68±8 | 362±69 | 4 |
| Beispiel 19 | | | | | | |
| Variante mit Wasser | n.b. | 1,27±0,33 | 6 | 61±3 | 300±108 | 6 |
| Variante mit Aceton | n.b. | 1,14±0,23 | 4 | | nicht bestimmt | |
| Beispiel 20 | n.b. | 1,01±0,02 | 5 | 51±19 | 290±47 | 3 |
| Beispiel 21 | n.b. | 1,19±0,41 | 8 | 65±11 | 378±33 | 5 |
| Beispiel 22 | n.b. | nicht bestimmt | | 80±23 | 336±83 | 4 |
| Beispiel 23 | n.b. | 1,26±0,46 | 4 | | nicht bestimmt | |
| Kontrolltiere | | | | | | |
| Beispiel 1–5 | n.b. | 0,095±0,061 | 124 | 75±24 | 117±79 | 36 |
| Beispiel 6–14 | 1,0 | 0,07±0,04 | 62 | 65±7 | 131±99 | 28 |
| Beispiel 15–23 | n.b. | 0,08±0,06 | 98 | 81±20 | 120±68 | 23 |

[*] Phospholipidfraktion

Auswertung der Verträglichkeit:

Die Verträglichkeit ist der Quotient aus Anzahl der überlebenden Tiere pro Gesamttierzahl (n = 10); der Wert 1,0 bedeutet entsprechend ein Überleben aller 10 Tiere. Die Substanz wird als verträglich bezeichnet, wenn mindestens 9 Tiere überleben.

**Patentansprüche**

1. Lungen-Surfactant,
erhältlich durch

(A)
(A1) Extraktion von zerkleinertem Lungengewebe oder eines wäßrigen, mit einer isotonischen Lösung eines Elektrolyts oder eines Mono-oder Disaccharids hergestellten Lungenextrakts mit einem nicht mit Wasser mischbaren Lipidlösungsmittel,
(A2) Abtrennung ungelöster Bestandteile aus dem Extrakt,
(A3) Abtrennung des Lipidlösungsmittels,
(A4) Extraktion mit einem wasserlöslichen Alkohol,
(A5) Abtrennung alkoholunlöslicher Bestandteile aus dem Extrakt
und

(A6) Abtrennung des Alkohols und Gewinnung des Wirkstoffs

oder
(B)
(B1) Abzentrifugieren der Zellfragmente aus einem wäßrigen, mit einer isotonischen Lösung eines Elektrolyts oder eines Mono-oder Disaccharids hergestellten Lungenex-trakts bei niederen relativen Zentrifugalbe-schleunigungen,
(B2) Inkontaktbringen des Extrakts mit einem unlöslichen Adsorbens,
(B3) Waschen des beladenen Adsorbens mit Wasser oder Aceton unter Entfernung von nicht adsorbiertem Material,
(B4) Desorption des Wirkstoffs mit einem organischen Lösungsmittel oder einem Lösungs-mittelgemisch
und
(B5) Abtrennung des bzw. der Lösungsmittel unter Gewinnung des Wirkstoffs.

2. Lungen-Surfactant nach Anspruch 1, gekennzeichnet durch
40–70% Phospholipide,
≤ 1,5% und vorzugsweise 0,5–1,0% Proteine,
10–40% Cholesterin und
5–30% Neutrallipide.

3. Lungen-Surfactant nach Anspruch 1 oder 2, erhältlich durch Ausrühren von zerkleinertem Lungengewebe mit der 2- bis 10-fachen Gewichtsmenge isotonischer Kochsalzlösung oder Lösungen von Mono- oder Disacchariden in Verfahren (A), Schritt (A1).

4. Lungen-Surfactant nach einem der Ansprüche 1 bis 3, erhältlich durch Extraktion von zerkleinertem Lungengewebe mit 0,5 bis 10 l Lipidlösungsmittel/kg Lungengewebe bzw. mit dem 0,1- bis 2-fachen Volumen an Lipidlösungsmittel in Verfahren (A), Schritt (A1).

5. Lungen-Surfactant nach einem der Ansprüche 1 bis 4, erhältlich durch Extraktion von zerkleinertem Lungengewebe mit 1,5 l Lipidlösungsmittel/kg Lungengewebe.

6. Lungen-Surfactant nach einem der Ansprüche 1 bis 5, erhältlich unter Verwendung von halogenierten Kohlenwasserstoffen als Lipidlösungsmittel in Verfahren (A), Schritt (A1).

7. Lungen-Surfactant nach Anspruch 6, erhältlich unter Verwendung von Dichlormethan, Trichlormethan und/oder Tetrachlormethan als Lipidlösungsmittel in Verfahren (A), Schritt (A1).

8. Lungen-Surfactant nach einem der Ansprüche 1 bis 5, erhältlich unter Verwendung von Petrolether als Lipidlösungsmittel in Verfahren (A), Schritt (A1).

9. Lungen-Surfactant nach einem der Ansprüche 1 bis 8, erhältlich unter Verwendung von Methanol und/oder Ethanol als wasserlöslichen Alkohol und Anwendung einer Extraktionstemperatur von −20 bis +60 °C in Verfahren (A), Schritt (A4).

10. Lungen-Surfactant nach Anspruch 9, erhältlich unter Anwendung einer Extraktionstemperatur von 10 bis 30 °C in Verfahren (A), Schritt (A4).

11. Lungen-Surfactant nach einem der Ansprüche 1 bis 10, erhältlich unter Wiederholung von Schritt (A4), Verfahren (A).

12. Lungen-Surfactant nach einem der Ansprüche 1 bis 11, erhältlich unter Einsatz des Alkohols in einer Menge von 1 bis 30 ml/g roher Wirkstoff in Verfahren (A), Schritt (A4).

13. Lungen-Surfactant nach Anspruch 12, erhältlich unter Einsatz des Alkohols in einer Menge von 2 bis 20 ml/g roher Wirkstoff in Verfahren (A), Schritt (A4).

14. Lungen-Surfactant nach einem der Ansprüche 1 bis 13, ferner gekennzeichnet durch Gehalt einer Phospholipidfraktion im Verhältnis 10:1 bis 1:2 und vorzugsweise 1:1, die erhalten ist durch
Lösen von 0,05 bis 2 g des nach Verfahren (A), Schritte (A1) bis (A6) erhaltenen Wirkstoffs pro ml eines Lipidlösungsmittels und anschließende
Fällung mit Aceton im Verhältnis 1:0,5 bis 1:10 und vorzugsweise 1:3 bis 1:5.

15. Lungen-Surfactant nach Anspruch 14, dadurch gekennzeichnet, daß die Phospholipidfraktion durch Fällung bei −20 bis +40 °C erhalten ist.

16. Lungen-Surfactant nach Anspruch 14 oder 15, dadurch gekennzeichnet, daß die Phospholipidfraktion durch Fällung bei 2 bis 10 °C erhalten ist.

17. Lungen-Surfactant nach Anspruch 1 oder 2, erhältlich unter Verwendung eines Adsorbens auf Kieselsäurebasis in Verfahren (B), Schritt (B2).

18. Lungen-Surfactant nach Anspruch 17, erhältlich unter Verwendung von aktiviertem Kieselgel oder Magnesiumsilicat als Adsorbens in Verfahren (B), Schritt (B2).

19. Lungen-Surfactant nach einem der Ansprüche 1, 2, 17 oder 18, erhältlich unter Verwendung eines Lösungsmittelgemischs aus Methylenchlorid oder Chloroform und Methanol im Verhältnis 2:1 in Verfahren (B), Schritt (B4).

20. Phospholipidfraktion des Lungen-Surfactants nach einem der Ansprüche 1 bis 13, 17, 18 oder 19, erhältlich durch
Lösen von 0,05 bis 2 g des nach Verfahren (A), Schritte (A1) bis (A6) erhaltenen Wirkstoffs pro ml eines Lipidlösungsmittels und anschließende
Fällung mit Aceton im Verhältnis 1:0,5 bis 1:10 und vorzugsweise 1:3 bis 1:5.

21. Phospholipidfraktion nach Anspruch 20, erhältlich durch Fällung bei −20 bis +40 °C.

22. Phospholipidfraktion nach Anspruch 20 oder 21, erhältlich durch Fällung bei 2 bis 10 °C.

23. Verfahren zur Herstellung des Lungen-Surfactants nach einem der Ansprüche 1 bis 19, gekennzeichnet durch

(A)
(A1) Extraktion von zerkleinertem Lungengewebe oder eines wäßrigen, mit einer isotonischen Lösung eines Elektrolyts oder eines Mono- oder Disaccharids hergestellten Lungenextrakts mit einem nicht mit Wasser mischbaren Lipidlösungsmittel,
(A2) Abtrennung ungelöster Bestandteile aus dem Extrakt,
(A3) Abtrennung des Lipidlösungsmittels,
(A4) Extraktion mit einem wasserlöslichen Alkohol,
(A5) Abtrennung alkoholunlöslicher Bestandteile aus dem Extrakt und
(A6) Abtrennung des Alkohols und Gewinnung des Wirkstoffs −
oder

(B)
(B1) Abzentrifugieren der Zellfragmente aus einem wäßrigen, mit einer isotonischen Lösung eines Elektrolyts oder eines Mono- oder Disaccharids hergestellten Lungenextrakts bei niederen relativen Zentrifugalbeschleunigungen,
(B2) Inkontaktbringen des Extrakts mit einem unlöslichen Adsorbens,
(B3) Waschen des beladenen Adsorbens mit Wasser oder Aceton unter Entfernung von nicht adsorbiertem Material,
(B4) Desorption des Wirkstoffs mit einem organischen Lösungsmittel oder einem Lösungsmittelgemisch und
(B5) Abtrennung des bzw. der Lösungsmittel unter Gewinnung des Wirkstoffs.

24. Verfahren nach Anspruch 23, gekennzeichnet durch Ausrühren von zerkleinertem Lungengewebe mit der 2- bis 10-fachen Gewichtsmenge isotonischer Kochsalzlösung oder Lösungen von Mono- oder Disacchariden in Verfahren (A), Schritt (A1).

25. Verfahren nach Anspruch 23 oder 24, gekennzeichnet durch Extraktion von zerkleinertem Lungengewebe mit 0,5 bis 10 l Lipidlösungsmittel/kg Lungengewebe bzw. mit dem 0,1- bis 2-fachen Volumen Lipidlösungsmittel in Verfahren (A), Schritt (A1).

26. Verfahren nach Anspruch 25, gekennzeichnet durch Extraktion von zerkleinertem Lungengewebe mit 1,5 l Lipidlösungsmittel/kg Lungengewebe in Verfahren (A), Schritt (A1).

27. Verfahren nach einem der Ansprüche 23 bis 26, gekennzeichnet durch Verwendung von halogenierten Kohlenwasserstoffen als Lipidlösungsmittel in Verfahren (A), Schritt (A1).

28. Verfahren nach Anspruch 27, gekennzeichnet durch Verwendung von Dichlormethan, Trichlormethan und/oder Tetrachlormethan als Lipidlösungsmittel in Verfahren (A), Schritt (A1).

29. Verfahren nach einem der Ansprüche 23 bis 26, gekennzeichnet durch Verwendung von Petrolether als Lipidlösungsmittel in Verfahren (A), Schritt (A1).

30. Verfahren nach einem der Ansprüche 23 bis 29, gekennzeichnet durch Verwendung von Methanol und/oder Ethanol als wasserlöslichen Alkohol und Anwendung einer Extraktionstemperatur von −20 bis +60 °C in Verfahren (A), Schritt (A4).

31. Verfahren nach Anspruch 30, gekennzeichnet durch Verwendung von Methanol und/oder Ethanol als wasserlöslichen Alkohol und Anwendung einer Extraktionstemperatur von 10 bis 30 °C in Verfahren (A), Schritt (A4).

32. Verfahren nach einem der Ansprüche 23 bis 31, gekennzeichnet durch Wiederholung des Schritts (A4) in Verfahren (A).

33. Verfahren nach einem der Ansprüche 23 bis 32, gekennzeichnet durch Einsatz des Alkohols in einer Menge von 1 bis 30 ml/g roher Wirkstoff in Verfahren (A), Schritt (A4).

34. Verfahren nach Anspruch 33, gekennzeichnet durch Einsatz des Alkohols in einer Menge von 2 bis 20 ml/g roher Wirkstoff in Verfahren (A), Schritt (A4).

35. Verfahren zur Herstellung des Lungen-Surfactants nach Anspruch 14, gekennzeichnet durch Zugabe einer Phospholipidfraktion im Verhältnis 10:1 bis 1:2 und vorzugsweise 1:1 zum nach Verfahren (A), Schritte (A1) bis (A6) erhaltenen Wirkstoff, wobei die Phospholipidfraktion hergestellt wird durch

Lösen von 0,05 bis 2 g des nach Verfahren (A), Schritte (A1) bis (A6) erhaltenen Wirkstoffs pro ml eines Lipidlösungsmittels und anschließende

Fällung mit Aceton im Verhältnis 1:0,5 bis 1:10 und vorzugsweise 1:3 bis 1:5.

36. Verfahren nach Anspruch 35, gekennzeichnet durch Zugabe einer Phospholipidfraktion, die durch Fällung bei −20 bis +40 °C erhalten ist.

37. Verfahren nach Anspruch 35, gekennzeichnet durch Zugabe einer Phospholipidfraktion, die durch Fällung bei 2 bis 10 °C erhalten ist.

38. Verfahren zur Herstellung des Lungen-Surfactants nach einem der Ansprüche 1 bis 19, gekennzeichnet durch Verwendung eines Adsorbens auf Kieselsäurebasis in Verfahren (B), Schritt (B2).

39. Verfahren nach Anspruch 38, gekennzeichnet durch Verwendung von aktiviertem Kieselgel oder Magnesiumsilicat als Adsorbens in Verfahren (B), Schritt (B2).

40. Verfahren nach einem der Ansprüche 23, 24, 38 oder 39, gekennzeichnet durch Verwendung eines Lösungsmittelgemischs aus Methylenchlorid oder Chloroform und Methanol im Verhältnis 2:1 in Verfahren (B), Schritt (B4).

41. Verfahren zur Herstellung der Phospholipidfraktion nach einem der Ansprüche 20 bis 22, gekennzeichnet durch

Lösen von 0,05 bis 2 g des nach Verfahren (A), Schritte (A1) bis (A6) erhaltenen Wirkstoffs pro ml eines Lipidlösungsmittels und anschließende

Fällung mit Aceton im Verhältnis 1:0,5 bis 1:10 und vorzugsweise 1:3 bis 1:5.

42. Verfahren nach Anspruch 41, gekennzeichnet durch Fällung bei −20 bis +40 °C.

43. Verfahren nach Anspruch 41 oder 42, gekennzeichnet durch Fällung bei 2 bis 10 °C.

44. Pharmazeutische Mittel, insbesondere zur Behandlung des durch Surfactantmangel bedingten Atemnotsyndroms, gekennzeichnet durch

das Lungen-Surfactant nach einem der Ansprüche 1 bis 19 und/oder die Phospholipidfraktion nach einem der Ansprüche 20 bis 22 als Wirkstoff neben üblichen Hilfs- und/oder Trägerstoffen.

45. Pharmazeutische Mittel nach Anspruch 44, gekennzeichnet durch eine zur Applikation über die Atemwege geeignete galenische Form.

46. Pharmazeutische Mittel nach Anspruch 44 oder 45, gekennzeichnet durch eine physiologische Trägerflüssigkeit und einen Gehalt an Wirkstoff bzw. Wirkstoffgemisch von 1 bis 500 mg/ml Trägerflüssigkeit.

47. Verfahren zur Herstellung der pharmazeutischen Mittel nach einem der Ansprüche 44 bis 46, gekennzeichnet durch

Lösen des Wirkstoffs bzw. Wirkstoffgemischs in einem Lösungsmittel,

gegebenenfalls Abtrennung unlöslicher Bestandteile,

Sterilfiltration,

Einengen im Vakuum zur Trockne,

Emulgieren in destilliertem Wasser und

Lyophilisierung.

48. Verfahren nach Anspruch 47, gekennzeichnet durch Verwendung von Ethanol oder Chloroform als Lösungsmittel.

## Claims

1. Lung surfactant, obtainable by

(A)

(A1) extraction of comminuted lung tissue or of an aqueous lung extract prepared with an isotonic solution of an electrolyte or of a mono- or disaccharide with a water-immiscible lipid solvent,

(A2) separating off of undissolved components from the extract,

(A3) separating off of the lipid solvent,

(A4) extraction with a water-soluble alcohol,

(A5) separating off of alcohol-insoluble components from the extract and

(A6) separating off of the alcohol and obtaining of the active material or

(B)

(B1) centrifuging off the cell fragments from an aqueous lung extract prepared with an isotonic solution of an electrolyte or of a mono- or disaccharide at relatively low centifugal accelerations,

(B2) bringing into contact of the extract with an insoluble adsorbent,

(B3) washing of laden adsorbent with water or acetone with removal of non-adsorbed material,

(B4) desorption of the active material with an organic solvent or a solvent mixture and

(B5) separating off of the solvent or solvents with obtaining of the active material.

2. Lung surfactant according to claim 1, characterised by

40–70% phospholipids,

≤ 1.5% and preferably 0.5–1.0% proteins,

10–40% cholesterol

5–30% neutral lipids.

3. Lung surfactant according to claim 1 or 2, obtainable by stirring up of comminuted lung tissue with the 2 to 10 fold amount by weight of isotonic common salt solution or solutions of mono- or disaccharides in process (A), step (A1).

4. Lung surfactant according to one of claims 1 to 3, obtainable by extraction of comminuted lung tissue with 0.5 to 10 l. of lipid solvent/kg. of lung tissue or with the 0.1 to 2 fold volume of lipid solvent in process (A), step (A1).

5. Lung surfactant according to one of claims 1 to 4, obtainable by extraction of comminuted lung tissue with 1.5 l. of lipid solvent/kg. of lung tissue.

6. Lung surfactant according to one of claims 1 to 5, obtainable with the use of halogenated hydrocarbons as lipid solvents in process (A), step (A1).

7. Lung surfactant according to claim 6, obtainable with the use of dichloromethane, trichloromethane and/or tetrachloromethane as lipid solvent in process (A), step (A1).

8. Lung surfactant according to one of claims 1 to 5, obtainable with the use of petroleum ether as lipid solvent in process (A), step (A1).

9. Lung surfactant according to one of claims 1 to 8, obtainable with the use of methanol and/or ethanol as water-soluble alcohol and use of an extraction temperature of $-20$ to $+60\,°C$. in process (A), step (A4).

10. Lung surfactant according to claim 9, obtainable with the use of an extraction temperature of 10 to $30\,°C$. in process (A), step (A4).

11. Lung surfactant according to one of claims 1 to 10, obtainable with the repetition of step (A4), process (A).

12. Lung surfactant according to one of claims 1 to 11, obtainable with the use of the alcohol in an amount of 1 to 30 mg./g. of crude active material in process (A), step (A4).

13. Lung surfactant according to claim 12, obtainable with the use of the alcohol in an amount of 2 to 20 ml./g. of crude active material in process (A), step (A4).

14. Lung surfactant according to one of claims 1 to 13, further characterised by a content of a phospholipid fraction in the ratio of 10:1 to 1:2 and preferably of 1:1 which is obtainable by dissolving of 0.05 to 2 g. of the active material obtained according to process (A), steps (A1) to (A6) per ml. of a lipid solvent and subsequent precipitating with acetone in the ratio of 1:0.5 to 1:10 and preferably of 1:3 to 1:5.

15. Lung surfactant according to claim 14, characterised in that the phospholipid fraction is obtained by precipitation at $-20$ to $+40\,°C$.

16. Lung surfactant according to claim 14 or 15, characterised in that the phospholipid fraction is obtained by precipitation at 2 to $10\,°C$.

17. Lung surfactant according to claim 1 or 2, obtainable with the use of an adsorbent based on silicic acid in process (B), step (B2).

18. Lung surfactant according to claim 17, obtainable with the use of activated silica gel or magnesium silicate as adsorbent in process (B), step (B2).

19. Lung surfactant according to one of claims 1, 2, 17 or 18, obtainable with the use of a solvent mixture of methylene chloride or chloroform and methanol in the ratio of 2:1 in process (B), step (B4).

20. Phospholipid fraction of the lung surfactant according to one of claims 1 to 13, 17, 18 or 19, obtainable by dissolving of 0.05 to 2 g. of the active material obtainable according to process (A), steps (A1) to (A6) per ml. of a lipid solvent and subsequent precipitation with acetone in the ratio of 1:0.5 to 1:10 and preferably of 1:3 to 1:5.

21. Phospholipid fraction according to claim 20, obtainable by precipitation at $-20$ to $+40\,°C$.

22. Phospholipid fraction according to claim 20 or 21, obtainable by precipitation at 2 to $10\,°C$.

23. Process for the preparation of the lung surfactant according to one of claims 1 to 19, characterised by

(A)

(A1) extraction of comminuted lung tissue or of an aqueous lung extract produced with an isotonic solution of an electrolyte or of a mono- or disaccharide with a water-immiscible lipid solvent,

(A2) separating off of undissolved components from the extract,

(A3) Separating off of the lipid solvent,

(A4) extraction with a water-soluble alcohol,

(A5) separating off of alcohol-insoluble components from the extract and

(A6) separating off of the alcohol and obtaining of the active material or

(B)

(B1) centrifuging off of the cell fragments from an aqueous lung extract prepared with an isotonic solution of an electrolyte or of a mono- or disaccharide at relatively low centrifugal accelerations,

(B2) bring into contact of the extract with an insoluble adsorbent,

(B3) washing of the laden adsorbent with water or acetone with removal of non-adsorbed material,

(B4) desorption of the active material with an organic solvent or a solvent mixture and

(B5) separating off of the solvent or solvents with obtaining of the active material.

24. Process according to claim 23, characterised by stirring up of comminuted lung tissue with the 2 to 10 fold amount by weight of isotonic common salt solution or solutions of mono- or disaccharides in process (A), step (A1).

25. Process according to claim 23 or 24, characterised by extraction of comminuted lung tissue with 0.5 to 10 l. of lipid solvent/kg. of lung tissue or with the 0.1 to 2 fold volume of lipid solvent in process (A), step (A1).

26. Process according to claim 25, characterised by extraction of comminuted lung tissue with 1.5 l. of lipid solvent/kg. of lung tissue in process (A), step (A1).

27. Process according to one of claims 23 to 26, characterised by use of a halogenated hydrocarbons as lipid solvents in process (A), step (A1).

28. Process according to claim 27, characterised by the use of dichloromethane, trichloromethane and/or tetrachloromethane as lipid solvent in process (A), step (A1).

29. Process according to one of claims 23 to 26, characterised by the use of petroleum ether as lipid solvent in process (A), step (A1).

30. Process according to one of claims 23 to 29, characterised by the use of methanol and/or ethanol as water-soluble alcohol and use of an extraction temperature of $-20$ to $+60\,^\circ$C. in process (A), step (A4).

31. Process according to claim 30, characterised by the use of methanol and/or ethanol as water-soluble alcohol and use of an extraction temperature of 10 to 30 °C. in process (A), step (A4).

32. Process according to one of claims 23 to 31, characterised by repetition of step (A4) in process (A).

33. Process according to one of claims 23 to 32, characterised by the use of the alcohol in an amount of 1 to 30 ml./g. of crude active material in process (A), step (A4).

34. Process according to claim 33, characterised by the use of the alcohol in an amount of 2 to 20 ml./g. of crude active material in process (A), step (A4).

35. Process for the preparation of the lung surfactant according to claim 14, characterised by the addition of a phospholipid fraction in the ratio of 10:1 to 1:2 and preferably of 1:1 to the active material obtained according to process (A), steps (A1) to (A6), whereby the phospholipid fraction is prepared by dissolving of 0.05 to 2 g. of the active material obtained according to process (A), steps (A1) to (A6) per ml. of a lipid solvent and subsequent precipitation with acetone in the ratio of 1:0.5 to 1:10 and preferably of 1:3 to 1:5.

36. Process according to claim 35, characterised by the addition of a phospholipid fraction which is obtained by precipitation at $-20$ to $+40\,^\circ$C.

37. Process according to claim 35, characterised by the addition of a phospholipid fraction which is obtained by precipitation at 2 to 10 °C.

38. Process for the preparation of the lung surfactant according to one of claims 1 to 19, characterised by the use of an adsorbent based on silicic acid in process (B), step (B2).

39. Process according to claim 38, characterised by the use of activated silica gel or magnesium silicate as adsorbent in process (B), step (B2).

40. Process according to one of claims 23, 24, 38 or 39, characterised by the use of a solvent mixture of methylene chloride or chloroform and methanol in the ratio of 2:1 in process (B), step (B4).

41. Process for the preparation of the phospholipid fraction according to one of claims 20 to 22, characterised by dissolving of 0.05 to 2 g. of the active material obtained according to process (A), steps (A1) to (A6) per ml. of a lipid solvent and subsequent precipitation with acetone in the ratio of 1:0.5 to 1:10 and preferably of 1:3 to 1:5.

42. Process according to claim 41, characterised by precipitation at $-20$ to $+40\,^\circ$C.

43. Process according to claim 41 or 42, characterised by precipitation at 2 to 10 °C.

44. Pharmaceutical agent, especially for the treatment of the shortage of breath syndrome caused by surfactant deficiency, characterised by the lung surfactant according to one of claims 1 to 19 and/or the phospholipid fraction according to one of claims 20 to 22 as active material, besides usual adjuvant and/or carrier materials.

45. Pharmaceutical agent according to claim 44, characterised by a galenic form suitable for administration via the respiratory path.

46. Pharmaceutical agent according to claim 44 or 45, characterised by a physiological carrier liquid and a content of active material or active material mixture of 1 to 500 mg./ml. of carrier liquid.

47. Process for the preparation of the pharmaceutical agent according to one of claims 44 to 46, characterised by dissolving of the active material or active material mixture in a solvent, possible separating off of insoluble components, sterile filtration, evaporation to dryness in a vacuum, emulsifying in distilled water and lyophilisation.

48. Process according to claim 47, characterised by the use of ethanol or chloroform as solvent.

**Revendications**

1. Tensioactif pulmonaire pouvant être obtenu par

(A)
(A1) extraction de tissu pulmonaire broyé ou d'un extrait de poumon aqueux préparé avec une solution isotonique d'un électrolyte ou d'un mono- ou disaccharide, à l'aide d'un solvant des lipides non miscible à l'eau,
(A2) séparation des constituants non dissous à partir de l'extrait,
(A3) séparation du solvant des lipides,
(A4) extraction avec un alcool hydrosoluble,
(A5) séparation des constituants insolubles dans l'alcool à partir de l'extrait et
(A6) séparation de l'alcool et obtention du principe actif ou par

(B)
(B1) séparation par centrifugation des fragments de cellules à partir d'un extrait de poumon aqueux préparé avec une solution isotonique d'un électrolyte ou d'un mono- ou disaccharide avec de faibles accélérations de centrifugation relatives,
(B2) mise en contact de l'extrait avec un adsorbant insoluble,
(B3) lavage de l'adsorbant chargé avec de l'eau ou de l'acétone en vue de l'élimination de matière non adsorbée,
(B4) désorption du principe actif avec un solvant organique ou un mélange de solvants et
(B5) séparation du ou des solvants en vue de l'obtention du principe actif.

2. Tensioactif pulmonaire selon la revendication 1, caractérisé par
40–70% de phospholipides,
$\leq$ 1,5% et, de préférence 0,5–1,0% de protéines,
10–40% de cholestérol et
5–30% de lipides neutres.

3. Tensioactif pulmonaire selon la revendication 1 ou 2, pouvant être obtenu par extraction sous agitation de tissu pulmonaire broyé avec 2 à 10 fois la quantité pondérale de solution de chlorure de sodium isotonique ou de solutions de mono- ou disaccharides dans le procédé (A), étape (A1).

4. Tensioactif pulmonaire selon l'une des revendications 1 à 3, pouvant être obtenu par extraction de tissu pulmonaire broyé avec 0,5 à 10 l de solvant des lipides/kg de tissu pulmonaire, respectivement avec 0,1 à 2 fois le volume de solvant des lipides dans le procédé (A), étape (A1).

5. Tensioactif pulmonaire selon l'une des revendications 1 à 4, pouvant être obtenu par extraction de tissu pulmonaire broyé avec 1,5 l de solvant des lipides/kg de tissu pulmonaire.

6. Tensioactif pulmonaire selon l'une des revendications 1 à 5, pouvant être obtenu par emploi d'hydrocarbures halogénés comme solvants des lipides dans le procédé (A), étape (A1).

7. Tensioactif pulmonaire selon la revendication 6, pouvant être obtenu par emploi de dichlorométhane, de trichlorométhane et/ou de tétrachlorométhane comme solvants des lipides dans le procédé (A), étape (A1).

8. Tensioactif pulmonaire selon l'une des revendications 1 à 5, pouvant être obtenu par emploi d'éther de pétrole comme solvant des lipides dans le procédé (A), étape (A1).

9. Tensioactif pulmonaire selon l'une des revendications 1 à 8, pouvant être obtenu par emploi de méthanol et/ou d'éthanol comme alcool hydrosoluble et par application d'une température d'extraction de –20 à +60 °C dans le procédé (A), étape (A4).

10. Tensioactif pulmonaire selon la revendication 9, pouvant être obtenu par application d'une température d'extraction de 10 à 30 °C dans le procédé (A), étape (A4).

11. Tensioactif pulmonaire selon l'une des revendications 1 à 10, pouvant être obtenu par répétition de l'étape (A4), procédé (A).

12. Tensioactif pulmonaire selon l'une des revendications 1 à 11, pouvant être obtenu par mise en œuvre de l'alcool en une quantité de 1 à 30 ml/g de principe actif brut dans le procédé (A), étape (A4).

13. Tensioactif pulmonaire selon la revendication 12, pouvant être obtenu par mise en œuvre de l'alcool en une quantité de 2 à 20 ml/g de principe actif brut dans le procédé (A), étape (A4).

14. Tensioactif pulmonaire selon l'une des revendications 1 à 13, caractérisé en outre par une teneur en une fraction phospholipidique dans le rapport 10:1 à 1:2 et, de préférence de 1:1, qui est obtenue par
dissolution de 0,05 à 2 g du principe actif obtenu selon le procédé (A), étapes (A1) à (A6), par ml d'un solvant des lipides et précipitation subséquente avec de l'acétone dans le rapport de 1:0,5 à 1:10 et, de préférence de 1:3 à 1:5.

15. Tensioactif pulmonaire selon la revendication 14, caractérisé en ce que la fraction phospholipidique est obtenue par précipitation entre –20 et +40 °C.

16. Tensioactif pulmonaire selon la revendication 14 ou 15, caractérisé en ce que la fraction phospholipidique est obtenue par précipitation entre 2 à 10 °C.

17. Tensioactif pulmonaire selon la revendication 1 ou 2, pouvant être obtenu par emploi d'un adsorbant à base d'acide silicique dans le procédé (B), étape (B2).

18. Tensioactif pulmonaire selon la revendication 17, pouvant être obtenu par emploi de gel de silice activé ou de silicate de magnésium comme adsorbant dans le procédé (B), étape (B2).

19. Tensioactif pulmonaire selon l'une des revendications 1, 2, 17 ou 18, pouvant être obtenu par emploi d'un mélange de solvants à partir de chlorure de méthylène ou de chloroforme ou de méthanol dans un rapport de 2:1 dans le procédé (B), étape (B4).

20. Fraction phospholipidique du tensioactif pulmonaire selon l'une des revendications 1 à 13, 17, 18 ou 19, pouvant être obtenue par

dissolution de 0,05 à 2 g du principe actif obtenu selon le procédé (A), étapes (A1) à (A6) par ml d'un solvant des lipides et

précipitation subséquente avec de l'acétone dans le rapport de 1:0,5 à 1:10 et, de préférence de 1:3 à 1:5.

21. Fraction phospholipidique selon la revendication 20, pouvant être obtenue par précipitation entre −20 et +40°C.

22. Fraction phospholipidique selon la revendication 20 ou 21, pouvant être obtenue par précipitation entre 2 et 10°C.

23. Procédé de préparation du tensioactif pulmonaire selon l'une des revendications 1 à 19, caractérisé par

(A)

(A1) extraction de tissu pulmonaire broyé ou d'un extrait de poumon aqueux préparé avec une solution isotonique d'un électrolyte ou d'un mono- ou disaccharide, à l'aide d'un solvant des lipides non miscible à l'eau,

(A2) séparation des constituants non dissous à partir de l'extrait,

(A3) séparation du solvant des lipides,

(A4) extraction avec un alcool hydrosoluble,

(A5) séparation des constituants insolubles dans l'alcool à partir de l'extrait et

(A6) séparation de l'alcool et obtention du principe actif ou par

(B)

(B1) séparation par centrifugation des fragments de cellules à partir d'un extrait de poumon aqueux préparé avec une solution isotonique d'un électrolyte ou d'un mono- ou disaccharide avec de faibles accélérations de centrifugation relatives,

(B2) mise en contact de l'extrait avec un adsorbant insoluble,

(B3) lavage de l'adsorbant chargé avec de l'eau ou de l'acétone en vue de l'élimination de matière non adsorbée,

(B4) désorption du principe actif avec un solvant organique ou un mélange de solvants et

(B5) séparation du ou des solvants en vue de l'obtention du principe actif.

24. Procédé selon la revendication 23, caractérisé par une extraction sous agitation de tissu pulmonaire broyé avec 2 à 10 fois la quantité pondérale de solution de chlorure de sodium isotonique ou de solutions de mono- ou disaccharides dans le procédé (A), étape (A1).

25. Procédé selon la revendication 23 ou 24, caractérisé par l'extraction de tissu pulmonaire broyé avec 0,5 à 10 l de solvant des lipides/kg de tissu pulmonaire, respectivement avec 0,1 à 2 fois le volume de solvant des lipides dans le procédé (A), étape (A1).

26. Procédé selon la revendication 25, caractérisé par l'extraction de tissu pulmonaire broyé avec 1,5 l de solvant des lipides/kg de tissu pulmonaire dans le procédé (A), étape (A1).

27. Procédé selon l'une des revendications 23 à 26, caractérisé par l'emploi d'hydrocarbures halogénés comme solvants des lipides dans le procédé (A), étape (A1).

28. Procédé selon la revendication 27, caractérisé par l'emploi de dichlorométhane, de trichlorométhane et/ou de tétrachlorométhane comme solvants des lipides dans le procédé (A), étape (A1).

29. Procédé selon l'une des revendications 23 à 26, caractérisé par l'emploi d'éther de pétrole comme solvant des lipides dans le procédé (A), étape (A1).

30. Procédé selon l'une des revendications 23 à 29, caractérisé par l'emploi de méthanol et/ou d'éthanol comme alcool hydrosoluble et par l'application d'une température d'extraction de −20 à +60°C dans le procédé (A), étape (A4).

31. Procédé selon la revendication 30, caractérisé par l'emploi de méthanol et/ou d'éthanol comme alcool hydrosoluble et par l'application d'une température d'extraction de 10 à 30°C dans le procédé (A), étape (A4).

32. Procédé selon l'une des revendications 23 à 31, caractérisé par la répétition de l'étape (A4) dans le procédé (A).

33. Procédé selon l'une des revendications 23 à 32, caractérisé par la mise en œuvre de l'alcool en une quantité de 1 à 30 ml/g de principe actif brut dans le procédé (A), étape (A4).

34. Procédé selon la revendication 33, caractérisé par la mise en œuvre de l'alcool en une quantité de 2 à 20 ml/g de principe actif brut dans le procédé (A), étape (A4).

35. Procédé de préparation du tensioactif pulmonaire selon la revendication 14, caractérisé par l'addition d'une fraction phospholipidique dans le rapport de 10:1 à 1:2 et, de préférence de 1:1, au principe actif obtenu selon le procédé (A), étapes (A1) à (A6), la fraction phospholipidique étant préparée par

dissolution de 0,05 à 2 g du principe actif obtenu selon le procédé (A), étapes (A1) à (A6) par ml d'un solvant des lipides et

précipitation consécutive avec de l'acétone dans le rapport de 1:0,5 à 1:10 et, de préférence de 1:3 à 1:5.

36. Procédé selon la revendication 35, caractérisé par l'addition d'une fraction phospholipidique qui est obtenue par précipitation entre −20 et +40°C.

37. Procédé selon la revendication 35, caractérisé par l'addition d'une fraction phospholipidique qui est obtenue par précipitation entre 2 et 10°C.

38. Procédé de préparation du tensioactif pulmonaire selon l'une des revendications 1 à 19, caractérisé par l'emploi d'un adsorbant à base d'acide silicique dans le procédé (B), étape (B2).

39. Procédé selon la revendication 38, caractérisé par l'emploi de gel de silice activé ou de silicate de magnésium comme adsorbant dans le procédé (B), étape (B2).

40. Procédé selon l'une des revendications 23, 24, 38 ou 39, caractérisé par l'emploi d'un mélange de solvants à partir de chlorure de méthylène ou de chloroforme ou de méthanol dans un rapport de 2:1 dans le procédé (B), étape (B4).

41. Procédé de préparation de la fraction phospholipidique selon l'une des revendications 20 à 22, caractérisé par

la dissolution de 0,05 à 2 g du principe actif obtenu selon le procédé (A), étapes (A1) à (A6) par ml d'un solvant des lipides et

précipitation subséquente avec de l'acétone dans le rapport de 1:0,5 à 1:10 et, de préférence de 1:3 à 1:5.

42. Procédé selon la revendication 41, caractérisé par une précipitation entre −20 et +40 °C.

43. Procédé selon la revendication 41 ou 42, caractérisé par une précipitation entre 2 et 10 °C.

44. Produits pharmaceutiques, notamment pour le traitement du syndrome dyspnéique lié au manque de tensioactif, caractérisés par le tensioactif pulmonaire selon l'une des revendications 1 à 18 et/ou la fraction phospholipidique selon l'une des revendications 20 à 22 en tant que principe actif, à côté des adjuvants et/ou des excipients usuels.

45. Produits pharmaceutiques selon la revendication 44, caractérisés par une forme galénique se prêtant à l'application par la voie respiratoire.

46. Produits pharmaceutiques selon la revendication 44 ou 45, caractérisés par un liquide vecteur physiologique et une teneur en principe actif ou en mélange de principes actifs de 1 à 500 mg par ml de liquide vecteur.

47. Procédé de préparation des produits pharmaceutiques selon l'une des revendications 44 à 46, caractérisé par la dissolution du principe actif, respect. du mélange de principes actifs dans un solvant, éventuellement par la séparation des constituants insolubles,

filtration stérile,

concentration sous vide jusqu'à siccité,

émulsification dans de l'eau distillée et

lyophilisation.

48. Procédé selon la revendication 47, caractérisé par l'emploi d'éthanol ou de chloroforme comme solvant.

## Fig.1

# Fig. 2

Oberflächenspannung (mN/m)

Fläche

# Fig.3

## Fig.4

# Fig.5

1. 0.06 ml Surfactant nach Beispiel 23
2. 0,07-0,10 ml Surfactant nach Beispiel 23

Fig. 6

Fig. 7

x = PEEP 5

Fig. 8

x - PEEP 5

1.→ 1,0 ml Surfactant nach Beispiel 22

2.→ 0,75ml Surfactant nach Beispiel 22

Fig. 9